# EUROPEAN PATENT SPECIFICATION

(11) **EP 1 530 469 B1**
(45) Date of publication and mention of the grant of the patent: **25.02.2009**
(21) Application number: 03749093.5
(22) Date of filing: 20.08.2003
(51) Int. Cl.: A61K 31/445, A61K 9/70, A61K 31/4468, A61P 25/36

(54) **TRANSDERMAL DOSAGE FORM COMPRISING AN ACTIVE AGENT AND A SALT AND FREE-BASE FORM OF AN ANTAGONIST**
TRANSDERMALE DOSIERUNGSFORM ENTHALTEND EINEN WIRKSTOFF UND EINEN ANTAGONISTEN IN FREIER BASE UND IN SALZ FORM
FORME POSOLOGIQUE TRANSDERMIQUE COMPRENANT UN PRINCIPE ACTIF, UN SEL ET UNE FORME DE BASE LIBRE D'UN ANTAGONISTE

(30) Priority: 20.08.2002 US 404980 P
(43) Date of publication of application: 18.05.2005
(73) Proprietor: EURO-CELTIQUE S.A., 1653 Luxembourg (LU)
(72) Inventor: SHEVCHUK, Ihor, Yonkers, NY 10710 (US); REIDENBERG, Bruce, Rye, NY 01580 (US)
(74) Representative: Maiwald, Walter
(86) International application number: PCT/US2003/026132
(87) International publication number: WO 2004/017941

(56) References cited:
- WO-A-99/32119
- WO-A-03/070191
- US-A- 5 236 714

## Description

### 1. FIELD OF THE INVENTION

The present invention relates to transdermal dosage forms useful for preventing or discouraging the abuse of an active pharmaceutical agent, such as an opioid. The present invention further relates to tamper-resistant transdermal dosage forms comprising an active pharmaceutical agent or a pharmaceutically acceptable salt thereof, and an antagonist of the active pharmaceutical agent, the antagonist comprising both a free base adverse agent and a pharmaceutically acceptable salt of the antagonist. In particular, the present invention relates to tamper-resistant transdermal dosage forms comprising an opioid or a pharmaceutically acceptable salt thereof, a free-base opioid antagonist, and a pharmaceutically acceptable salt of an opioid antagonist.

### 2. BACKGROUND OF THE INVENTION

Transdermal dosage forms are convenient for delivering many different therapeutically effective agents, including but not limited to analgesics, such as opioid analgesics. Typical opioid analgesics include, but are not limited to, fentanyl, buprenorpline, etorphines, and other high potency narcotics. Other therapeutically effective agents that can be delivered using a transdermal dosage form include, but are not limited to, anti-emetics (for example, scopolamine), cardiovascular agents (for example, nitrates and clonidine), hormones (for example, estrogen and testosterone), nicotine, vitamins and dietary supplements.

The most common transdermal dosage form is a diffusion-driven transdermal system (particularly in the form of a patch) using either a fluid reservoir or a drug-in-adhesive matrix system. Other transdermal dosage forms include, but are not limited to, topical gels, lotions, ointments, transmucosal systems and devices, and iontophoretic (electrical-diffusion) delivery systems. See, for example, U.S. Patent No. 4,626,539 to Aungst et al*.,* which discloses a pharmaceutical composition purportedly useful for transdermal delivery of an opioid to a mammalian circulation system; U.S. Patent No. 4,806,341 to Chien et al*.,* which discloses transdermal-absorption dosage units comprising a backing layer, an adhesive polymer layer, and an adjoining layer of a solid polymer matrix containing a morphinan narcotic analgesic or antagonist and skin penetration enhancers; and U.S. Patent No. 5,069,909 to Sharma et al*.,* which discloses methods for sustained administration of buprenorphine using transdermal delivery from a laminated composite patch.

Transdermal dosage forms are particularly useful for timed-release and sustained-release of active agents. However, many dosage forms, and particularly those intended for timed and sustained release of active agents, contain large amounts of active agents, often in an amount that is many times the actual dose absorbed or delivered. Additionally, users sometimes remove the patch prior to the expiration of the recommended use period. Thus, there is often a significant amount of the active agent remaining in the dosage form after use and removal of the device by the user. Both the unused dosage form and the portion of active agent that remains in the dosage form after use are potentially subject to intentional or inadvertent abuse or misuse, particularly if the active agent is a narcotic or a controlled substance. For example, incompletely used dosage forms containing excess opioids can be tampered with by chewing or by extraction by a drug abuser. Even careful disposal of used dosage forms might not be completely effective for preventing abuse, particularly in cases of incomplete or partial compliance.

U.S. Patent No. 5,830,497 to Yamanaka et al*.* discloses a medicated plaster composition as a dosage form for percutaneous absorption, the composition containing either (1) a basic drug and an acidic substance; (2) a salt of a basic drug and a basic substance; or (3) a basic drug and a salt of a basic drug. The patent does not address the problem of abuse of the active drug, and does not address the use of antagonists to prevent such abuse.

U.S. Patent No. 5,804,215 to Cubbage et al*.* discloses a disposal system for a transdermal patch containing a medicament, such as nicotine, comprising an adhesive coated, flexible, tear-resistant substrate. The used transdermal patch allegedly adheres to the substrate in order to encapsulate and deter access to the transdermal patch. Rubber-based adhesives are disclosed as being preferred.

U.S. Patent No. 5,149,538 to Granger et al*.* discloses a transdermal patch comprising an opioid and an antagonist substance that are separated by an impermeable barrier. The antagonist substance is purportedly releasable from the patch upon being ingested or substantially immersed in a solvent.

U.S. Patent No. 5,236,714 to Lee et al*.* discloses a composition comprising an abusable substance and an antagonist for the abusable substance. A mixture of oraliy and parenterally active antagonists can allegedly be utilized. The patent does not address the problem of extraction using different solvent systems, and does not disclose the use of multiple antagonists having differing solubilities.

The inadvertent misuse or abuse of transdermal dosage forms remains a significant health problem. Thus, there remains a need in the art for improved transdermal dosage forms that are effective for preventing abuse yet useful for delivering a therapeutic agent, such as an opioid or a pharmaceutically acceptable salt thereof.

### 3. SUMMARY OF THE INVENTION

In one embodiment, the present invention is directed to transdermal dosage forms comprising an active agent, a pharmaceutically acceptable salt of an antagonist and an antagonist in the form of a free base.

In another embodiment, the present invention is directed to transdermal dosage forms comprising an active agent, a pharmaceutically acceptable salt of an antagonist and an antagonist in the form of a free base, in an amount sufficient to inhibit at least one biological effect of the active agent.

In another embodiment, the present invention is directed to a transdermal dosage form comprising an analgesically effective amount of an opioid or a pharmaceutically acceptable salt thereof (hereinafter "Opioid"), an opioid antagonist in the form of a free base (hereinafter "Antagonist Free Base"), and a pharmaceutically acceptable salt of an opioid antagonist (hereinafter "Antagonist Salt"), in an amount sufficient to inhibit the euphoric effect of the Opioid.

The invention is further directed to methods for treating or preventing pain in an animal comprising contacting the skin of an animal in need thereof with a transdermal dosage form comprising an analgesically effective amount of an Opioid, an Antagonist Free Base and an Antagonist Salt, in an amount sufficient to inhibit the euphoric effect of the Opioid, and wherein the contacting is for an amount of time sufficient to treat or prevent pain.

The present invention may be understood more fully by reference to the following figures, detailed description and example, which are intended to exemplify non-limiting embodiments of the invention.

### 4. BRIEF DESCRIPTION OF THE DRAWINGS

FIG. 1 is a schematic cross-section of a reservoir-type transdermal dosage form.
FIG. 2 is a schematic cross-section of a polymer-matrix transdermal dosage form.
FIG. 3 is a schematic cross-section of a drug-in-adhesive transdermal dosage form.
FIG. 3A is a schematic cross-section of an alternative drug-in-adhesive transdermal dosage form.
FIGS. 4 and 5 are graphs depicting an amount of extracted Opioid or Antagonist vs. time.

### 5. DETAILED DESCRIPTION OF THE INVENTION

The present invention relates to a transdermal dosage form useful for the transdermal administration of an active agent, such as an Opioid, to an animal, and to methods for treating or preventing pain in an animal comprising contacting the skin of an animal in need thereof with the transdermal dosage form of the invention for an amount of time sufficient to treat the patient, such as to treat or prevent pain. In one embodiment, the transdermal dosage form of the present invention comprises an active agent, an adverse agent in the form of a free base and an adverse agent salt. In one embodiment, an adverse agent salt is present in an amount sufficient to inhibit at least one biological effect of an active agent when it and the adverse agent in the form of a free base are administered (by means such as, buccally, nasally, sublingually, parenterally, rectally, and/or vaginally) to an animal, more preferably, a human. In another embodiment, an adverse agent in the form of a free base is present in an amount sufficient to inhibit at least one biological effect of an active agent when it and the adverse agent salt are administered (by means such as, buccally, nasally, parenterally, rectally, and/or vaginally) to an animal, more preferably, a human. In a further embodiment, an adverse agent in the form of a free base and an adverse agent salt are provided in a total amount sufficient to inhibit at least one biological effect of an active agent when it, the adverse agent in the form of a free base and the adverse agent salt are administered (by means such as, buccally, nasally, sublingually, parenterally, rectally, and/or vaginally) to an animal, more preferably, a human. In a further embodiment, an adverse agent in the form of a free base and an adverse agent salt are provided in a total amount sufficient to inhibit at least one biological effect of an active agent when the transdermal dosage form is subjected to abuse or misuse. In another embodiment, an Antagonist Salt is present in an amount sufficient to inhibit the euphoric effect of an Opioid when it and the Antagonist Free Base are administered (by means such as, buccally, nasally, sublingually, parenterally, rectally, and/or vaginally) to an animal, more preferably, a human. In another embodiment, an Antagonist Salt is present in an amount sufficient to inhibit the euphoric effect of an Opioid when it and the Antagonist Free Base are administered (by means such as, buccally, nasally, parenterally, rectally, and/or vaginally) to an animal, more preferably, a human. In a further embodiment, an Antagonist Free Base and an Antagonist Salt are provided in a total amount sufficient to inhibit the euphoric effect of an Opioid when it, the Antagonist Free Base and the Antagonist Salt are administered (by means such as, buccally, nasally, sublingually, parenterally, rectally, and/or vaginally) to an animal, more preferably, a human. In a further embodiment, an Antagonist Free Base and an Antagonist Salt are provided in a total amount sufficient to inhibit the euphoric effect of an Opioid when the transdermal dosage form is subjected to abuse or misuse.

When contacted with an animal's skin the transdermal dosage form allows for the transdermal administration of an Opioid, but either (a) allows for the transdermal administration of only an amount of an Antagonist Free Base or Antagonist Salt (each being an "Antagonist") that is ineffective for inhibiting the analgesic effect of the Opioid, or (b) does not allow for the transdermal administration of an Antagonist. But if the transdermal dosage form of the invention is used to deliver an Opioid via a route other than transdermal, such as buccal, nasal, oral, parenteral, rectal and/or vaginal, or if the transdermal dosage form is subjected to abuse or misuse, then one or both of the Antagonists inhibit the euphoric effect of the Opioid. Preferably, the transdermal dosage form will inhibit the euphoric effect of an Opioid if the device is used other than transdermally whether before or after the device is used by an animal for treating or preventing pain.

The transdermal dosage form of the invention is tamper-resistant in that if an abuser attempts to extract or separate an Opioid from the transdermal dosage form, and self-administer the Opioid via another route, such as, but not limited to, oral, parenteral, nasal, or buccal, rectal or vaginal, i.e., a route of administration that can result in a quick euphoric rush, also known as the "burst," that abusers can prefer, the abuser would self-administer an amount of an Antagonist along with the Opioid, the amount of Antagonist being effective to inhibit the euphoric effect of the Opioid.

For example, if an abuser tries to extract an Opioid from the transdermal dosage form by placing it in a solvent, including saliva, then an amount of an Antagonist would also be extracted, providing a mixture of the Opioid and the Antagonist. The Antagonist Free Base generally exhibits greater solubility, and in one embodiment bioavailability, in non-aqueous solvents than in aqueous solvents, while an Antagonist Salt generally exhibits greater solubility and, in one embodiment, bioavailability in aqueous solvents than in non-aqueous solvents. Thus, if an abuser attempts to extract an Opioid from the transdermal dosage form by use of a non-aqueous solvent such as an ether or an alcohol, an Antagonist Free Base would be extracted along with the Opioid, providing a mixture of the Opioid and Antagonist: Alternatively, if an abuser attempts to extract an Opioid from the transdermal dosage form by use of an aqueous solvent such as water or saliva, an Antagonist Salt would be extracted along with the Opioid, providing a mixture of the Opioid and Antagonist.

If a mixture of an Opioid and Antagonist is administered via a route other than the intended transdermal route, then at least one Antagonist would exert its antagonistic effect to inhibit the euphoric effect of the Opioid.

The invention further relates to methods for treating or preventing pain in an animal, comprising transdermally administering to an animal in need thereof an analgesically effective amount of an Opioid, using a transdermal dosage form of the invention.

### 5.1 DEFINITIONS

The phrase "transdermal dosage form," as used herein means any device that when contacted with an animal's skin, can transdermally deliver an effective amount of any biologically active agent, such as a pharmaceutical agent, such as an opioid, through the animal's skin.

As used herein the terms "dosage form" and "delivery device" are synonymous and interchangeable.

Any reference herein to any pharmaceutical agent including, but not limited to, an active agent, an adverse agent, an opioid agonist or an opioid antagonist, shall, unless otherwise stated, include any pharmaceutically acceptable form of such pharmaceutical agent, such as the free form, any pharmaceutically acceptable salt form, any pharmaceutically acceptable base form, any pharmaceutically acceptable hydrate, any pharmaceutically acceptable solvate, any stereoisomer, any optical isomer, as well as any prodrug of such pharmaceutical agent and any pharmaceutically active analog of such pharmaceutical agent, and mixtures of any of the foregoing.

As used herein, the phrase "active agent" refers to a pharmaceutical agent, therapeutic agent, drug, and/or agonist that causes a biological effect when absorbed in sufficient quantity into the blood stream of a patient.

As used herein, the phrase "adverse agent" refers to a pharmaceutical agent, drug, and/or antagonist that partially or completely prevents, negates, diminishes, delays or reverses at least one biological effect of the active agent present in the dosage form, *e.g*. euphoric effect, or produces one or more unpleasant physiological reactions, *e.g*., vomiting, nausea, diarrhea, bad taste, when absorbed in sufficient amount into the blood stream of a patient or patient.

As used herein, the terms "opioid" or "opioid agonist" refer to an active agent which exhibits opium- or morphine-like properties when absorbed in sufficient amounts into the bloodstream of a patient or patient. Opioid agonists bind, optionally stereospecifically, to any one or more of several subspecies of opioid receptors and produce agonist activity.

As used herein, the phrase "opioid antagonist" refers to an adverse agent that either partially or completely prevents, negates, diminishes, delays or reverses at least one biological effect of an opioid agonist, *e.g*., euphoric effect, when absorbed in sufficient amounts into the blood stream of a patient or patient.

The phrase "pharmaceutically acceptable salt," as used herein, is a salt formed from an acid and the basic nitrogen group of an opioid. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, glubionate and pamoate (*i.e.,* 1,1'-methylene-bis-(2-hydroxy-3-naphthoate)) salts. The term "pharmaceutically acceptable salt" also refers to a salt prepared from an opioid having an acidic functional group, such as a carboxylic acid or sulfonic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N, N,-dimethyl-N-(2-hydroxyethyl)amine, or tri-(2-hydroxyethyl)amine; N-methyl-D-glucamine; and amino acids such as arginine, lysine, and the like.

The term "animal," as used herein, includes, but is not limited to, a cow, monkey, horse, sheep, pig, chicken, turkey, quail, cat, dog, mouse, rat, rabbit, and guinea pig, and is more preferably a mammal, and most, preferably a human.

The phrase "treatment of pain" or "treating pain," as used herein, includes amelioration of pain or the cessation of pain in an animal.

The phrase "prevention of pain" or "preventing pain," as used herein, includes the avoidance of the onset of pain in an animal.

### 5.2 THE TRANSDERMAL DOSAGE FORM

Any device known to those skilled in the art for transdermally delivering a therapeutic agent, particularly an Opioid, to an animal can be used for the transdermal dosage form of the invention. For example, the transdermal dosage form can be a reservoir-type transdermal dosage form, a polymer-matrix type transdermal dosage form, or a drug-in-adhesive type transdermal dosage form (*See, e.g.,* H.S. Tan et al., Pressure Sensitive Adhesives for Transdermal Drug Delivery Systems, in PSTT 2(2):60-79 (1999). The transdermal dosage form is designed so that when contacted with the animal's skin, an analgesically effective amount of the active therapeutic agent, such as an Opioid, is transdermally administered to the animal. But when contacted with an animal's skin, an Antagonist Free Base and an Antagonist Salt either remain in the transdermal dosage form and are not administered to the animal or are administered to the animal in an amount insufficient to inhibit the analgesic effect of the active agent.

A reservoir-type transdermal dosage form typically comprises a reservoir, usually a liquid, located between an impermeable backing film and a rate-controlling membrane that is covered with a pressure-sensitive adhesive skin-contacting layer. The reservoir, which can be a solution or a dispersion, comprises an active agent, such as an Opioid, Antagonist Free Base and Antagonist Salt. The transdermal dosage form is supported by the impermeable backing material and the adhesive surface is protected by a release liner. Suitable backing materials include, but are not limited to, polyethylene, polyethylene derivatives, polypropylene, polyesters, polyurethanes, nylon fibers and natural fibers. Suitable release liners include, but are not limited to conventional release liners comprising a sheet material such as a polyester web, a polyethylene web, a polystyrene web or a polyethylene-coated paper coated with a suitable fluoropolymer or silicone based coating. To administer an active agent, such as an Opioid, the release liner is removed to expose the pressure-sensitive adhesive and the pressure-sensitive adhesive is contacted with the skin. The active agent is permeable to the rate-controlling membrane, and penetrates through it and the adhesive, contacts the skin, and then penetrates the skin. The delivery rate of an active agent, such as an Opioid, is partially controlled by the rate that the active agent penetrates the rate-controlling membrane. Preferably, the pressure-sensitive adhesive does not adversely affect the delivery rate of and does not chemically react with the active agent. The delivery rate is such that an analgesically effective amount of an active agent, such as an Opioid, is delivered to the animal. In contrast to an active agent, such as an Opioid, an Antagonist, which can be present anywhere in the reservoir, preferably does not penetrate the rate-controlling membrane or, if it does, in an amount insufficient to inhibit the analgesic effect of the active agent.

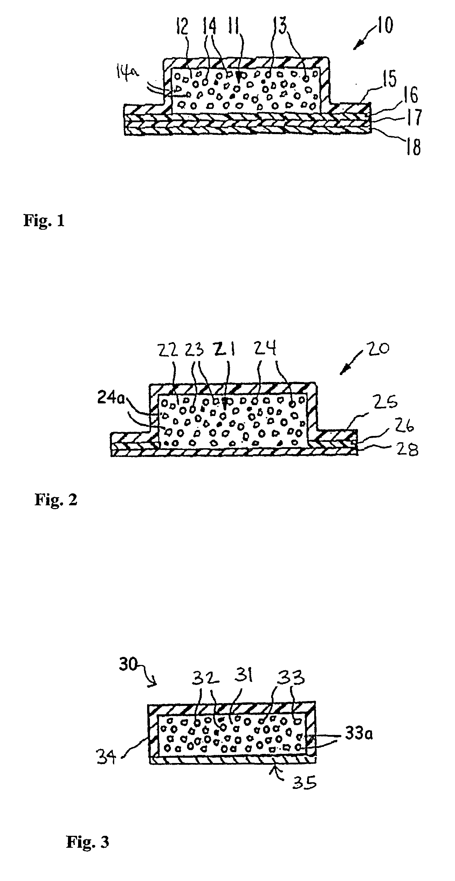

FIG. 1 depicts one embodiment of a reservoir-type transdermal dosage form. The transdermal dosage form 10 comprises a reservoir 11, typically in the form of a solution or a dispersion 12, having dispersed therein an active agent, such as an Opioid 13, an Antagonist Free Base 14, and an Antagonist Salt, 14a. The reservoir 11 is disposed between an impermeable backing film 15, a rate-controlling membrane 16, and a pressure-sensitive adhesive 17. A release liner 18 is applied to the pressure-sensitive adhesive layer 17, and is removed prior to use. In one embodiment, the Opioid 13, and the Antagonists 14, 14a, of which at least one is an Antagonist Free Base 14, and at least one is an Antagonist Salt 14a, are dispersed throughout the reservoir, although uniform dispersion is not necessary.

A variation of the reservoir-type transdermal dosage form is the polymer-matrix design. In the polymer-matrix design, an active agent, such as an Opioid, and the Antagonists are dispersed in a polymer matrix that partially controls the delivery rate of the active agent. Similar to the liquid-reservoir design, the polymer-matrix reservoir is supported on a impermeable backing layer. Rather than having a continuous adhesive layer, however, the polymer-matrix design usually includes a peripheral ring of adhesive located around the edge of the patch. A release liner protects the adhesive surface and the surface of the polymer matrix. To administer an active agent, such as an Opioid, the release liner is removed to expose the polymer matrix and the ring of pressure-sensitive adhesive, and the device is contacted with the skin. The ring of adhesive holds the device against the skin so that the polymer matrix directly contacts the skin. When the polymer matrix is contacted with the skin, an active agent, such as an Opioid, diffuses out of the polymer matrix, contacts the animal's skin, and penetrates the skin. The delivery rate of the Antagonists is partially controlled by the rate of diffusion of the Antagonists out of the polymer matrix. The delivery rate is such that an analgesically effective amount of an active agent, such as an Opioid, is delivered to the animal. The Antagonists, which may be present anywhere in the polymer matrix, on the other hand, either do not diffuse out of the polymer matrix or, if diffusion takes place, in an amount insufficient to inhibit the analgesic effect of the active agent.

FIG. 2 depicts a typical polymer-matrix transdermal dosage form embodiment of the invention. The transdermal dosage form 20 comprises a reservoir 21 in the form of a polymer matrix 22, having dispersed therein an active agent, such as an Opioid 23, an Antagonist Free Base 24, and an Antagonist Salt 24a. In one embodiment, an Opioid 23, and the Antagonists 24, 24a, are dispersed throughout the polymer matrix, although uniform dispersion is not necessary. The polymer matrix 21 is supported on an impermeable backing layer 25 and has a peripheral ring of adhesive 26 located around the edge of the patch. A release liner 28 is applied to the peripheral ring of adhesive 26 and polymer matrix 22 and is removed prior to use.

The drug-in-adhesive type transdermal dosage form comprises an active agent, such as an Opioid, an Antagonist Free Base and an Antagonist Salt dispersed directly in a pressure-sensitive adhesive matrix. The adhesive matrix is typically supported on the topside with an impermeable backing film and on the side that faces the skin with an impermeable release liner. To administer an active agent, such as an Opioid, the release liner is removed to expose the adhesive matrix, and the device is contacted with the skin. The adhesive matrix functions to adhere the device to the skin and, typically, to control the delivery rate of an active agent, such as an Opioid. Similar to the polymer-matrix design, the drub in-adhesive design allows an active agent to diffuse out of the adhesive matrix, contact the animal's skin, and penetrate the skin. The delivery rate of an active agent, such as an Opioid, is partially determined by the rate of diffusion of the active agent out of the adhesive matrix. The delivery rate is such that an analgesically effective amount of an active agent, such as an Opioid is delivered to the animal. The Antagonists, which can be present anywhere in the adhesive matrix, on the other hand, do not diffuse out of the adhesive matrix or do so in an amount insufficient to inhibit the analgesic effect of an active agent.

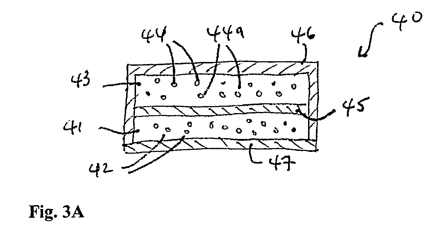

FIG. 3 depicts a typical drug-in-adhesive transdermal dosage form embodiment of the invention. The transdermal dosage form 30 comprises an adhesive matrix 31 having dispersed there through an active agent, such as an Opioid 32, Antagonist Free Base 33, and Antagonist Salt 33a. In one embodiment, an Opioid 32, and the Antagonists 33,33a, are dispersed throughout the polymer matrix, although uniform dispersion is not necessary. The adhesive matrix 31 is supported on an impermeable backing layer 34 and has an impermeable release liner 35 on the side that faces the skin which is removed prior to use.

FIG. 3A depicts another drug-in-adhesive transdermal dosage form embodiment of the invention. The transdermal dosage form 40 comprises a first adhesive matrix 41 having dispersed therethrough an active agent, such as an Opioid 42, and a second adhesive matrix 43 having dispersed therethrough an Antagonist Free Base and an Antagonist Salt, 44, 44a. The first and second layers are separated by a barrier layer, 45. Preferably, an Opioid and the Antagonists are dispersed throughout the adhesive matrix, although uniform dispersion is not necessary. The adhesive matrix system is supported on an impermeable overlay backing layer 46 and has an impermeable release liner 47 on the side that faces the skin which is removed prior to use.

The reservoir type, polymer matrix type, and the drug-in-adhesive type transdermal delivery systems are well-known to those skilled in the art (*See, e.g.,* H.S.Tan et al., Pressure Sensitive adhesives for Transdermal Drug Delivery Systems, in PSTT 2(2):60-79 (1999)), the contents of which are expressly incorporated herein by reference).

Any rate-controlling membrane known to those skilled in the art can be used in the transdermal dosage form of the invention. In one embodiment, the membrane does not allow any, or any detectable amount, of an Antagonist to penetrate therethrough, particularly in those instances in which an Antagonist can penetrate an animal's skin. Suitable materials for the rate-controlling membranes include, but are not limited to, polyethylene; polypropylene; ethylene/propylene copolymers; ethylene/ethylacrylate copolymers; ethylene/vinyl acetate copolymers; polyacrylates; polymethacrylates; silicone elastomers; medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example polysiloxane-polycarbonate copolymers, polysiloxane-polyethyleneoxidecopolymers, polysiloxane-polymethacrylate copolymers, polysiloxane-alkylene copolymers (*e.g*., polysiloxane-ethylene copolymers), polysiloxane-alkylenesilane copolymers (*e.g*., polysiloxaneethylenesilane copolymers), and the like; cellulose polymers, for example, methyl or ethyl cellulose, hydroxypropyl methyl cellulose, and cellulose esters; polycarbonates; polytetrafluoroethylene; starches; gelatin; natural and synthetic gums; any other natural or synthetic polymer or fiber; and combinations thereof.

The backing layer can be any suitable material that is impermeable to the contents of the reservoir compartment, the polymer matrix, or the adhesive matrix. Suitable materials for backing films are well known to those skilled in the art and include, but are not limited to, occlusive polymers such as polyurethane, polyesters such as poly(ethylene phthalate), polyether amide, copolyester, polyisobutylene, polyesters, high and low density polyethylene, polypropylene, polyvinylchloride, metal foils, and metal foil laminates of suitable polymer films.

Suitable materials for the polymer matrix are well known to those skilled in the art and include, but are not limited to, polyethylene; polypropylene; ethylene/propylene copolymers; ethylene/ethylacrylate copolymers; ethylene/vinyl acetate copolymers; silicone elastomers, especially the medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example, polysiloxane-polycarbonate copolymers, polysiloxane-polyethyleneoxide copolymers, polysiloxane-polymethacrylate copolymers, polysiloxane-alkylene copolymers (*e.g*., polysiloxane-ethylene copolymers), polysiloxane-alkylenesilane copolymers (*e.g*., polysiloxaneethylenesilane copolymers), and the like; cellulose polymers, for example methyl or ethyl cellulose, hydroxypropyl methyl cellulose, and cellulose esters; polycarbonates; polytetrafluoroethylene; and combinations thereof. In one embodiment, the polymer matrix has a glass-transition temperature below room temperature. The polymer can, but need not necessarily, have a degree of crystallinity at room temperature. Cross-linking monomeric units or sites can be incorporated into the polymers. For example, cross-linking monomers can be incorporated into polyacrylate polymers. The cross-linking monomers provide sites for cross-linking the polymer matrix after microdispersing the active agent, such as an Opioid, and an adverse agent, such as an Antagonist into the polymer. Known cross-linking monomers for polyacrylate polymers include, but are not limited to, polymethacrylic esters of polyols such as butylene diacrylate and dimethacrylate, trimethylol propane trimethacrylate, and the like. Other monomers that provide cross-linking sites include allyl acrylate, allyl methacrylate, diallyl maleate, and the like. In one embodiment the polymer matrix does not allow any, or any detectable amount, of an Antagonist to diffuse out of it, particularly in those instances in which the Antagonists can penetrate an animal's skin.

Suitable materials for the pressure-sensitive adhesive matrix are well known to those skilled in the art and include, but are not limited to, polyisobutylenes, polysiloxanes, and polyacrylate copolymers (polyacrylic esters), natural rubber/karaya gum-based adhesives, hydrogels, hydrophilic polymers, and polyurethanes such as those described in H.S. Tan et al., Pressure Sensitive Adhesives for Transdermal Drug Delivery Systems, in PSTT 2(2):60-79 (1999), the disclosure of which is incorporated herein by reference. The adhesive may further comprise modifying monomers, tackifiers, plasticizers, fillers, waxes, oils, and other additives to impart the desired adhesive properties. *Id.* In addition, one skilled in the art can readily achieve desired adhesives properties by the incorporation of materials such as initiators, crosslinkers and comonomers. In one embodiment the pressure-sensitive adhesive matrix does not allow any, or any detectable amount, of an Antagonist to diffuse out it, particularly in those instances in which the Antagonist can penetrate an animal's skin.

Preferred pressure-sensitive adhesives for use in the dosage forms of the invention include acrylates, polyisobutylenes, silicone polymers, and mixtures thereof. Examples of useful polyisobutylene pressure-sensitive adhesives are described in U.S. Pat. No. 5,985,317 (Venkateshwaran et al.), the disclosure of which is incorporated herein by reference. Examples of useful acrylate and silicone polymer pressure-sensitive adhesives, and mixtures thereof, are described in U.S. Pat. No. 5,474,783 (Miranda et al.), the disclosure of which is incorporated herein by reference.

Generally, the size of the device of can vary from about 1 cm² to greater than 200 cm² and typically are between about 5-50 cm². Methods for manufacturing transdermal dosage forms are well known to those skilled in the art.

Examples of devices useful in the transdermal dosage forms and methods of the invention include, but are not limited to, those described in U.S. Patent Nos. 4,806,341; 5,069,909; 5,236,714; 5,240,711; 5,902,603; 5,718,914; 5,968,547; 6,162,456; and 6,344,212, the disclosures of which are herein incorporated by reference.

The transdermal dosage form can optionally comprise one or more penetration enhancers, which increase the rate at which an active agent, such as an Opioid, penetrates through the animal's skin. In one embodiment, the penetration enhancer does not enhance the penetration of an Antagonist through the skin. In another embodiment, the penetration enhancer penetrates the rate-controlling membrane or diffuses out of the polymer matrix or adhesive matrix so that it can contact the animal's skin and improve penetration of an active agent, such as an Opioid through the animal's skin. Suitable penetration enhancers for use in the transdermal dosage forms and methods of the invention include, but are not limited, C₂-C₄ alcohols such as ethanol and isopropanol, polyethylene glycol monolaurate, diethyl glycol monomethyl ether, polyethylene glycol-3-lauramide, dimethyl lauramide, dimethyl isosorbide, sorbitan trioleate, fatty acids, esters of fatty acids having from about 10 to about 20 carbon atoms, monoglycerides or mixtures of monoglycerides of fatty acids having a total monoesters content of at least about 51 % where the monoesters are those with from 10 to 20 carbon atoms, and mixtures of mono-, di- and tri-glycerides of fatty acids. Suitable fatty, acids include, but are not limited to lauric acid, myristic acid, stearic acid, oleic acid, linoleic acid and palmitic acid. Monoglyceride permeation enhancers include glycerol monooleate, glycerol monolaurate, and glycerol monolinoleate, for example. Examples of penetration enhancers useful in the methods of the invention include, but are not limited to those described in U.S. Patents Nos. 3,472,931; 3,527,864; 3,896,238; 3,903,256; 3,952,099; 3,989,816; 4,046,886; 4,130,643; 4,130,667; 4,299,826; 4,335,115; 4,343,798; 4,379,454; 4,405,616; 4,746,515; 4,316,893; 4,405,616; 4,060,084, 4,379,454; 4,560,553 4,863,952; 4,863,970; 4,879,275; 4,940,586; 4,960,771; 4,973,468; 5,066,648; 5,164,406; 5,227,169; 5,229,130; 5,238,933; 5,308,625; 4,326,566; 5,378,730; 5,420,106; 5,641,504 5,716,638; 5,750,137; 5,785,991; 5,837,289; 5,834,468; 5,882,676; 5,912,009; 5,952,000; 6,004,578; and Idson, J. Pharm. Sci. 64(b6):901-924 (1975), the disclosures of which are herein incorporated by reference..

### 5.3 ACTIVE AGENTS

The transdermal dosage form can comprise any pharmacologically active agent that is capable of inducing a desired biological or pharmacological effect, which may include, but is not limited to, (1) affecting a living process; (2) having a prophylactic effect on an animal and preventing an undesired effect, such as preventing an infection; (3) alleviating a condition caused by, or a symptom of, a disease, *e.g*., pain or inflammation; and/or (4) alleviating, reducing, or eliminating a disease, condition, or symptom from the animal. The effect of the active agent may be local, such as for providing an anaesthetic effect, or it may be systemic or a combination thereof General categories of active agents can, in one embodiment, include, but are not limited to: opioids; ACE inhibitors; adenohypophoseal hormones; adrenergic neuron blocking agents; adrenocortical steroids; inhibitors of the biosynthesis of adrenocortical steroids; alpha-adrenergic agonists; alpha-adrenergic antagonists; selective alpha-two-adrenergic agonists; androgens; anti-addictive agents; antiandrogens; antiinfectives, such as antibiotics, antimicrobials, and antiviral agents; analgesics and analgesic combinations; anorexics; antihelminthics; antiarthritics; antiasthmatic agents; anticonvulsants; antidepressants; antidiabetic agents; antidiarrheals; antiemetic and prokinetic agents; antiepileptic agents; antiestrogens; antifungal agents; antihistamines; antiinflammatory agents; antimigraine preparations; antimuscarinic agents; antinauseants; antineoplastics; antiparasitic agents; antiparkinsonism drugs; antiplatelet agents; antiprogestins; antipruritics; antipsychotics; antipyretics; antispasmodics; anticholinergics; antithyroid agents; antitussives; azaspirodecanediones; sympathomimetics; xanthine derivatives; cardiovascular preparations, including potassium and calcium channel blockers, alpha blockers, beta blockers, and antiarrhythmics; antihypertensives; diuretics and antidiuretics; vasodilators, including general coronary, peripheral, and cerebral; central nervous system stimulants; vasoconstrictors; cough and cold preparations, including decongestants; hormones, such as estradiol and other steroids, including corticosteroids; hypnotics; immunosuppressives; muscle relaxants; parasympatholytics; psychostimulants; sedatives; tranquilizers; nicotine and acid addition salts thereof; benzodiazepines; barbiturates; benzothiadiazides; beta-adrenergic agonists; beta-adrenergic antagonists; selective beta-one-adrenergic antagonists; selective beta-two-adrenergic antagonists; bile salts; agents affecting volume and composition of body fluids; butyrophenones; agents affecting calcification; catecholamines; cholinergic agonists; cholinesterase reactivators; dermatological agents; diphenylbutylpiperidines; ergot alkaloids; ganglionic blocking agents; hydantoins; agents for control of gastric acidity and treatment of peptic ulcers; hematopoietic agents; histamines; 5-hydroxytryptamine antagonists; drugs for the treatment of hyperlipiproteinemia; laxatives; methylxanthines; monoamine oxidase inhibitors; neuromuscular blocking agents; organic nitrates; pancreatic enzymes; phenothiazines; prostaglandins; retinoids; agents for spasticity and acute muscle spasms; succinimides; thioxanthines; thrombolytic agents; thyroid agents; inhibitors of tubular transport of organic compounds; drugs affecting uterine motility; vitamins; and the like; or a combination thereof.

The transdermal dosage form can comprise an active component that may include, but is not limited to, flurogestone acetate, hydroxyprogesterone, hydroxyprogesterone acetate, hydroxyprogesterone caproate, medroxy-progesterone acetate, norethindrone, norethindrone acetate, norethisterone, norethynodrel, desogestrel, 3-keto desogestrel, gestadene, levonorgestrel, estradiol, estradiol benzoate, estradiol valerate, estradiol cyprionate, estradiol decanoate, estradiol acetate, ethynyl estradiol, estriol, estrone, mestranol, betamethasone, betamethasone acetate, cortisone, hydrocortisone, hydrocortisone acetate, corticosterone, fluocinolone acetonide, prednisolone, prednisone, triamcinolone, aldosterone, androsterone, testosterone, methyl testosterone, or a combination thereof.

The transdermal dosage form can comprise an active component that may include, but is not limited to: a) a corticosteroid, *e.g*., cortisone, hydrocortisone, prednisolone, beclomethasone propionate, dexamethasone, betamethasone, flumethasone, triamcinolone, triamcinolone acetonide, fluocinolone, fluocinolone acetonide, fluocinolone acetate, clobetasol propionate, or the like, or a combination thereof; b) an analgesic anti-inflammatory agent, *e.g*., acetaminophen, mefenamic acid, flufenamic acid, indomethacin, diclofenac, diclofenac sodium, alclofenac, ibufenac, oxyphenbutazone, phenylbutazone, ibuprofen, flurbiprofen, ketoprofen, salicylic acid, methylsalicylate, acetylsalicylic acid, 1-menthol, camphor, slindac, tolmetin sodium, naproxen, fenbufen, or the like, or a combination thereof; c) a hypnotic sedative, *e.g.,* phenobarbital, amobarbital, cyclobarbital, lorazepam, haloperidol, or the like, or a combination thereof; d) a tranquilizer, *e.g*., fulphenazine, thioridazine, diazepam, flurazepam, chlorpromazine, or the like, or a combination thereof; e) an antihypertensive, *e.g*., clonidine, clonidine hydrochloride, bopinidol, timolol, pindolol, propranolol, propranolol hydrochloride, bupranolol, indenolol, bucumolol, nifedipine, bunitrolol, or the like, or a combination thereof; f) a hypotensive diuretic, *e.g*., bendroflumethiazide, polythiazide, methylchlorthiazide, trichlormethiazide, cyclopenthiazide, benzyl hydrochlorothiazide, hydrochlorothiazide, bumetanide, or the like, or a combination thereof; g) an antibiotic, *e.g.,* penicillin, tetracycline, oxytetracycline, metacycline, doxycycline, minocycline, fradiomycin sulfate, erythromycin, chloramphenicol, or the like, or a combination thereof; h) an anesthetic, *e.g*., lydocaine, benzocaine, ethylaminobenzoate, or the like, or a combination thereof; i) another analgesic, *e.g*., acetylsalicylic acid, choline magnesium trisalicylate, acetaminophen, ibuprofen, fenoprofen, diflusinal, naproxen and the like; j) an antipruritic agent, *e.g*., bisabolol, oil of chamomile, chamazulene, allantoin, D-panthenol, glycyrrhetenic acid, a corticosteroid, an antihistamines and the like; k) an antimicrobial agent, *e.g*., methyl hydroxybenzoate, propyl hydroxybenzoate, chlorocresol, benzalkonium chlorides, nitrofurazone, nystatin, sulfacetamide, clotriamazole, or the like, or a combination thereof; 1) an antifungal agent, *e.g.,* pentamycin, amphotericin B, pyrrol nitrin, clotrimazole, or the like, or a combination thereof; m) a vitamin, *e.g*., vitamin A, ergocalciferol, cholecalciferol, octotriamine, riboflavin butyric acid ester, or the like, or a combination thereof; n) an antiepileptic, *e.g*., nitrazepam, meprobamate, clonazepam, or the like, or a combination thereof; o) an antihistamine, *e.g*., diphenhydramine hydrochloride, chlorpheniramine, diphenylimidazole, or the like, or a combination thereof; p) an antitussive, *e.g*., dextromethorphan, terbutaline, ephedrine, ephedrine hydrochloride, or the like, or a combination thereof; q) a sex hormone, *e.g*., progesterone, estradiol, estriol, estrone, or the like, or a combination thereof; r) an antidepressant, *e.g*., doxepin; s) a vasodilator, *e.g*., nitroglycerin, isosorbide nitrate, nitroglycol, pentaerythritol tetranitrate, dipyridamole, or the like, or a combination thereof; t) another drug, *e.g*., 5-fluorouracil, dihydroergotamine, desmopressin, digoxin, methoclopramide, domperidone, scopolamine, scopolamine hydrochloride, or the like, or a combination thereof; or the like; or a combination thereof.

The amount of active agent present in the transdermal dosage form will depend in part on the specific active agent, the type of device, the materials used in the device, and the duration for which the active agent will be delivered to the animal. The amount of an active agent present in the transdermal dosage form, however, typically ranges from about 0.01 to about 50 mg/cm², preferably from about 0.05 to about 15 mg/cm², and more preferably from about 0.05 to about 5.0 mg/cm². It is well within the purview of one skilled in the art to readily determine the amount of an active agent needed for a particular indication.

### 5.4 OPIOIDS

Any Opioid can be used in the transdermal dosage form of the present invention. Useful Opioids include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, pharmaceutically acceptable salts thereof and mixtures of any two or more thereof.

In certain embodiments, the Opioid is hydrocodone, morphine, hydromorphone, oxycodone, codeine, levorphanol, meperidine, methadone, oxymorphone, buprenorphine, fentanyl, dipipanone, heroin, tramadol, etorpline, dihydroetorphine, butorphanol, levorphanol, pharmaceutically acceptable salts thereof, and mixtures of any two or more thereof. In one embodiment, the Opioid is oxycodone, hydrocodone, fentanyl, buprenorphine, or a pharmaceutically acceptable salt thereof.

In one embodiment, especially for passive patches, an Opioid is in free-base form. For patches of the invention that use iontophoresis to facilitate penetration of the skin by an Opioid, however, a pharmaceutically acceptable salt of the Opioid is preferred.

The analgesically effective amount of Opioid present in the transdermal dosage form will depend in part on the specific Opioid, the type of device, the materials used in the device, and the duration for which the Opioid will be delivered to the animal. The analgesically effective amount of an Opioid present in the transdermal dosage form, however, typically ranges from about 0.01 to about 50 mg/cm², preferably from about 0.05 to about 15 mg/cm², and more preferably from about 0.05 to about 5.0 mg/cm². It is well within the purview of one skilled in the art to readily determine the analgesically effective amount of an Opioid needed for a particular indication.

### 5.5 ADVERSE AGENT

The adverse agent can be any pharmaceutical active agent which at least partially reduces or blocks at least one biological effect of an active agent or which creates an unpleasant effect when absorbed into an animal's or patient's blood stream. Examples of adverse agents include, but are not limited to, antagonists of any active agent. When an opioid agonist is used as the active agent in the dosage form of the present invention, an opioid antagonist can be used as the adverse agent. Likewise, when a benzodiazepine is used as the active agent in the dosage form of the present invention, a benzodiazepine antagonist can be used as the adverse agent. When a barbiturate is used as an active agent in the dosage form of the present invention, a barbiturate antagonist can be used as the adverse agent. When an amphetamine is used as an active agent in the dosage form of the present invention, an amphetamine antagonist can be used as the adverse agent. When the active agent is toxic when dosed above its normal therapeutic range, i.e., when there is a significant potential for an overdose, then an antidote of the toxic active agent can be used as the adverse agent.

Benzodiazepine antagonists that can be used as the adverse agent of the present invention include, but are not limited to, flumazenil.

Barbiturate antagonists which can be used as the adverse agent of the present invention include, but are not limited to, amphetamines.

Stimulant antagonists that can be used as the adverse agent of the present invention include, but are not limited to, benzodiazepines.

In another embodiment of the present invention, the adverse agent is an agent that causes an undesired physiological reaction, such as emesis. This type of adverse agent can be used with any kind of therapeutic agent including an opioid, a benzodiazepine, a barbiturate, or a stimulant. Examples of emetic agents suitable for use as the adverse agent in the present invention includes any drug that safely and effectively induces vomiting after administration including, but not limited to, ipecac and apomorphine.

### 5.6 OPIOID ANTAGONIST

Opioid antagonists that can be used in the transdermal dosage forms and methods of the invention include, but are not limited to, cyclazocine, naloxone, naltrexone, nalmefene, nalbuphine, nalorphine, cyclazacine, levallorphan, pharmaceutically acceptable salts thereof, and mixtures thereof. In certain embodiments, the Antagonist is nalmefene, naloxone, naltrexone, or a pharmaceutically acceptable salt thereof. The Antagonist Free Base and the Antagonist Salt can be based on the same or different antagonist; for example, the Antagonist Free Base and the Antagonist Salt can be naloxone and naloxone HCl, respectively.

In one embodiment, useful Antagonist Salts include salts formed from an acid and the basic nitrogen group of a free-base form of an antagonist. Examples of Antagonist Salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, glubionate and pamoate (i.e., 1,1'-methylene-bis-(2-hydroxy-3-naphthoate)) salts.

Other Antagonist Salts include salts prepared from an Antagonist having an acidic functional group, such as a carboxylic acid or sulfonic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N, N,-dimethyl-N-(2-hydroxyethyl)amine, or tri-(2-hydroxyethyl)amine; N-methyl-D-glucamine; and amino acids such as arginine, lysine, and the like.

The amount of the Antagonists present in the transdermal dosage form and sufficient to inhibit the euphoric effect of an active agent, such as an Opioid, depends in part on the particular Antagonists used in the device. Typically, the weight ratio of active agent, such as an Opioid, to the total amount of the Antagonists in the transdermal dosage form ranges from about 15:1 to about 1:5, more preferably from about 12:1 to about 4:1. Illustrative Antagonist combinations include:
naloxone HCl: naltrexone free base, having in one embodiment a weight ratio being from about 5:1 to about 1:5, and in another embodiment from about 1:1 to about 1:5;
naloxone free base: naltrexone HCl, having in one embodiment a weight ratio being from about 5:1 to about 1:5, and in another embodiment from about 1:5 to about 1:1;
naloxone HCl: nalmefene free base, having in one embodiment a weight ratio being from about 5:1 to about 1:5, and in another embodiment from about 1:1 to about 1:5; and
naltrexone HCl: nalmefene free base, having in one embodiment a weight ratio being from about 5:1 to about 1:5, and in another embodiment from about 1:1 to about 1:5.

In one embodiment the Opioid is fentanyl. Illustrative fentanyl:Antagonist combinations include:
fentanyl:naloxone HCl:naltrexone free base, having in one embodiment a weight ratio of fentanyl:Antagonist (total amount) combination being from about 10:1 to about 1:5, and in another embodiment from about 10:1 to about 4:1;
fentanyl:naloxone free base:naltrexone HCl, having in one embodiment a weight ratio of fentanyl:Antagonist (total amount) being from about 10:1 to about 1:5, and in another embodiment from about 10:1 to about 4:1;
fentanyl:naloxone HCl:nalmefene free base, having in one embodiment a weight ratio of fentanyl:Antagonist (total amount) being from about 15:1 to about 1:5, and in another embodiment from about 12:1 to about 4:1; and
fentanyl:naltrexone HCl:nalmefene free base, having in one embodiment a weight ratio of fentanyl:Antagonist (total amount) being from about 15:1 to about 1:5, and in another embodiment from about 12:1 to about 4:1.

One skilled in the art can readily determine the amount of Antagonists needed for a particular indication.

### 5.7 METHODS FOR TREATING OR PREVENTING PAIN

In accordance with one embodiment of the invention, the transdermal dosage form of the invention can be used to administer to an animal, preferably a mammal, more preferably a human, an analgesically effective amount of an active agent, such as an Opioid, for the treatment or prevention of pain. The transdermal dosage form can be used to treat or prevent acute or chronic pain. For example, the transdermal dosage form can be used for, but is not limited to, treating or preventing cancer pain, central pain, labor pain, myocardial infarction pain, pancreatic pain, colic pain, post-operative pain, headache pain, muscle pain, bone pain, and pain associated with intensive care.

According to the methods of the invention the transdermal dosage form is contacted with the skin of the animal, and an active agent, such as an Opioid, is released by the transdermal dosage form and becomes absorbed through the skin of the animal. Once absorbed into the animal, an active agent, such as an Opioid, is provided in an analgesically effective amount. The transdermal dosage form can provide sustained and continuous delivery of an analgesically effective amount of an active agent. In one embodiment, the transdermal dosage form of the invention maintains a level of an active agent, such as an Opioid, in the bloodstream of the animal between about 0.1 to about 100 nanograms of active agent per milliliter of blood plasma for about 16 hours to about 7 days, in another embodiment about 16 hours to about 72 hours, and in a further embodiment at least about 24 hours. In another embodiment, the transdermal dosage form of the invention maintains a level of an active agent, such as an Opioid, in the bloodstream of the animal sufficient to achieve an analgesic effect and, accordingly, treat or prevent pain for a period of time of between about 15 minutes to about 7 days, in another embodiment about 1 hour to about 72 hours, and in yet another embodiment at least about 24 hours.

The transdermal dosage forms of the invention are useful for treating or preventing pain in an animal comprising contacting the skin of an animal in need thereof with a transdermal dosage form of the invention for an amount of time sufficient to treat or prevent pain, for example from about 10 minutes to about 72 hours. In one embodiment, the amount of time is from about 30 minutes to about 24 hours.

### 5.8 KITS

The present invention is also directed to a kit comprising at least one dosage form of the invention. In one embodiment, the dosage form is present in a container, *e.g*., a bottle or box. In another embodiment, the kit further comprises a set of instructions directing the use of the dosage form to treat a patient, *e.g*., for pain. In one embodiment, the instructions may be a printed label affixed to or printed on the container. In another embodiment, the instructions may comprise a printed sheet inserted into the container or into the packaging which contains the container. The instructions may also state that the dosage form and/or its usage are designed to reduce abuse, misuse or diversion of the dosage form.

The following examples are set forth to assist in understanding the invention and should not, of course, be construed as specifically limiting the invention described and claimed herein. Such variations of the invention, including the substitution of all equivalents now known or later developed, which would be within the purview of those skilled in the art, and changes in formulation or minor changes in experimental design, are to be considered to fall within the scope of the invention incorporated herein.

### 6. EXAMPLES

The following examples are prophetic examples which are believed to be functional either as stated or with minor modifications as known to one skilled in the art. The examples serve to illustrate, rather than limit, the scope of the present invention.

### Example 1

A transdermal dosage form according to Figure 3A was prepared, with the first adhesive layer comprising 15 mg/cm² fentanyl free base in adhesive. The second adhesive layer comprised 15 mg/cm² nalmefene HCl in adhesive. Several 2cm² portions of the device were die-cut, the protective liner removed, the portions immobilized and added to testing vessels. The amount of fentanyl free base and of nalmefene HCl extractable from the device (and thus potentially available for abuse) in some cases contained within the transdermal disposal device, was determined through extraction using the following solvents: phosphate-buffered saline ("PBS"), alcohol/water mixture and ethyl ether. 15 mL aliquots of solvent were used in each extraction. The PBS solution was prepared by combining equal amounts of a 0.1M phosphate buffer (pH 6.5) and a 0.5M solution of sodium chloride. The alcohol/water mixture was prepared by mixing 75 parts absolute ethanol with 25 parts water.

The test sample vials were placed on a laboratory roller and agitated at 20 RPM. Several 1000 µL portions were extracted at 5-minute intervals and assayed using liquid chromatography. An assay for both fentanyl free base and nalmefene HCl was performed using liquid chromatography.

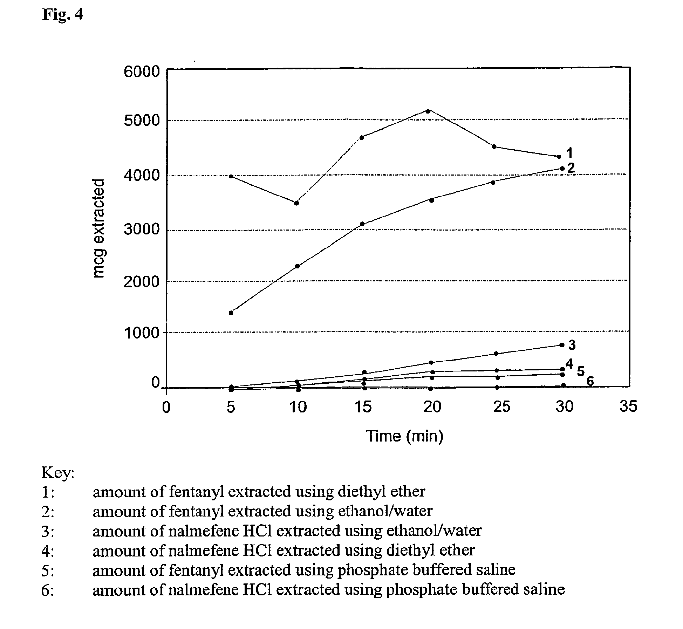

The results of the assay for fentanyl free base are shown in Table 1, and the results of the assay for nalmefene HCl are shown in Table 2. Figure 4 shows the amounts (microgram/minute) of fentanyl free base and nalmefene HCl extracted by PBS, alcohol/water mixture and diethyl ether, respectively, from the device.

**Table 1**

| EXTRACTION DATA FOR FENTANYL | | | |
|---|---|---|---|
| Time (minutes) | SOLVENT | | |
| | PBS (µg) | Ethanol/Water (µg) | Diethyl Ether (µg) |
| 5 | 0 | 1436 | 3949 |
| 10 | 132 | 2286 | 3492 |
| 15 | 158 | 3058 | 4674 |
| 20 | 198 | 3537 | 5196 |
| 25 | 222 | 3882 | 4508 |
| 30 | 256 | 4097 | 4339 |

**Table 2**

| EXTRACTION DATA FOR NALMEFENE HCL | | | |
|---|---|---|---|
| Time (minutes) | SOLVENT | | |
| | PBS (µg) | Ethanol/Water (µg) | Diethyl Ether (µg) |
| 5 | 0 | 38 | 0 |
| 10 | 0 | 140 | 52 |
| 15 | 4 | 251 | 193 |
| 20 | 5 | 480 | 314 |
| 25 | 14 | 650 | 318 |
| 30 | 15 | 834 | 353 |

### Example 2

A transdermal dosage form according to Figure 3A was prepared, with the first adhesive layer comprising 15 mg/cm² fentanyl free base in adhesive. The second adhesive layer comprised approximately equal amounts of naltrexone and nalmefene HCl in adhesive, at a total loading of 15 mg/cm². Several 2cm² portions of the device were die-cut, the protective liner removed, the portions immobilized and added to testing vessels. The amount of fentanyl free base, nalmefene HCl and naltrexone extractable from the device (and thus potentially available for abuse) in some cases contained within the transdermal disposal system, was determined through extraction using the following solvents: PBS, alcohol/water mixture and diethyl ether. 15 mL aliquots of solvent were used in each extraction. The PBS solution was prepared by combining equal amounts of a 0.1M phosphate buffer (pH 6.5) and a 0.5M solution of sodium chloride. The alcohol/water mixture was prepared by mixing 75 parts absolute ethanol with 25 parts water.

The test sample vials were placed on a laboratory roller and agitated at 20 RPM. Several 1000µL portions were extracted at 5-minute intervals and assayed using liquid chromatography. An assay for fentanyl free base, nalmefene HCl and naltrexone was performed using liquid chromatography.

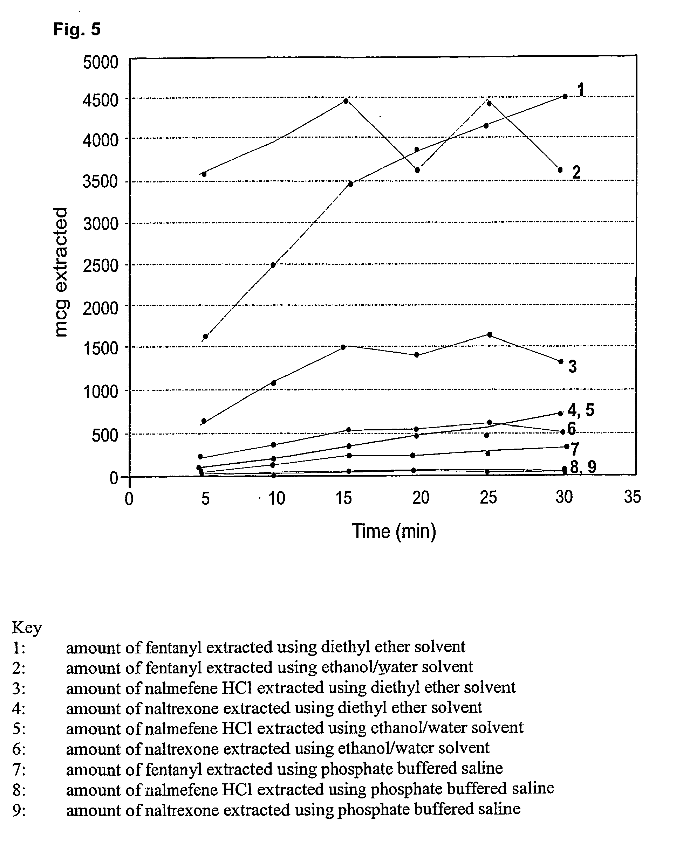

The results of the assay for fentanyl free base, nalmefene HCl and naltrexone are shown in Tables 3, 4 and 5, respectively. Figure 5 shows the amounts (microgram/minute) of fentanyl free base, nalmefene. HCl and naltrexone extracted by PBS, alcohol/water mixture and diethyl ether, respectively, from the device.

**Table 3**

| EXTRACTION DATA FOR FENTANYL | | | |
|---|---|---|---|
| Time(minutes) | SOLVENT | | |
| | PBS (µg) | Ethanol/Water (µg) | Diethyl Ether (µg) |
| 5 | 0 | 1591 | 3565 |
| 10 | 118 | 2489 | 3978 |
| 15 | 190 | 3439 | 4476 |
| 20 | 227 | 3893 | 3657 |
| 25 | 277 | 4206 | 4471 |
| 30 | 306 | 4513 | 3646 |

**Table 4**

| EXTRACTION DATA FOR NALMEFENE HCL | | | |
|---|---|---|---|
| Time (minutes) | SOLVENT | | |
| | PBS (µg) | Ethanol/Water (µg) | Diethyl Ether (µg) |
| 5 | 0 | 48 | 596 |
| 10 | 6 | 179 | 1092 |
| 15 | 9 | 334 | 1514 |
| 20 | 9 | 469 | 1425 |
| 25 | 26 | 568 | 1649 |
| 30 | 35 | 721 | 1346 |

**Table 5**

| EXTRACTION DATA FOR NALTREXONE | | | |
|---|---|---|---|
| Time (minutes) | SOLVENT | | |
| | PBS (µg) | Ethanol/Water (µg) | Diethyl Ether (µg) |
| 5 | 0 | 50 | 204 |
| 10 | 8 | 177 | 386 |
| 15 | 10 | 331 | 556 |
| 20 | 22 | 471 | 537 |
| 25 | 28 | 577 | 637 |
| 30 | 38 | 735 | 534 |

### Example 3

Table 6 shows the data obtained after 30 minutes for the patches tested in Examples 1 and 2.

**Table 6**

| EXTRACTION DATA AT 30 MINUTES | | | | |
|---|---|---|---|---|
| | | PBS (µg) | Ethanol/Water (µg) | Diethyl Ether (µg) |
| Example 1 | Fentanyl | 256 | 4097 | 4339 |
| | Nalmefene HCl | 15 | 834 | 353 |
| | Fentanyl:Antagonist Ratio | 17.0 | 4.9 | 12.3 |
| Example 2 | Fentanyl | 306 | 4513 | 3646 |
| | Nalmefene HCl | 35 | 721 | 1346 |
| | Naltrexone | 38 | 735 | 534 |
| | Fentanyl:Antagonist Ratio | 4.2 | 3.1 | 1.9 |

The ratios recited are the amount of extracted fentanyl divided by the total amount of extracted antagonist.

As shown in Table 6, extraction of an illustrative transdermal dosage form of the invention results in a mixture of an Opioid and Antagonist. The ratio of extracted Opioid to total extracted Antagonist in a patch having an Opioid, Antagonist Free Base and Antagonist Salt is lower than that of a transdermal device having only an Opioid and Antagonist Salt. Because extraction of the illustrative transdermal dosage form provides a lower ratio of extracted Opioid to extracted Antagonists relative to a device that contains an Opioid and an Antagonist Salt only, transdermal dosage forms of the invention are advantageous in that they are useful for discouraging or preventing abuse of a pharmaceutical agent, such as an Opioid.

### Example 4

Fentanyl free base (5.0 g) is added to ethanol (20 g) with mixing. Naloxone HCl (2.5g) is added to water (50g) and propylene glycol (10g) with mixing until dissolved. To the naloxone HCl solution is added naltrexone (2.5g) in ethanol (10 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9702, 3M Company) is coated with a skin-contact adhesive (Solutia 737), and the adhesive protected by a removable release liner (SCOTCHPAK 1022, 3M Company). A backing film (COTRAN 9701, 3M Company) is laminated to the membrane with a partial opening to allow for vertical filling with reservoir vehicle. After filling with reservoir vehicle to a loading of 5.0 mg/cm² fentanyl, the opening is laminated to produce a heat sealed reservoir-type transdermal dosage form. The reservoir contains a ratio of 1 part fentanyl to 1 part total Antagonist, with the ratio of antagonists being 1 part naloxone HCl to 1 part naltrexone. The transdermal dosage form is useful for treating or preventing pain in an animal, while preventing or discouraging the abuse of the Opioid.

### Example 5

Fentanyl free base (5.0 g) is added to ethanol (25 g) with mixing. Naloxone HCl (0.25g) is added to water (44g) and propylene glycol (5g) with mixing until dissolved. To the naloxone HCl solution is added naltrexone (0.25g) in ethanol (20 g). The resulting solution is combined with the fentanyl solution to form the reservoir vehicle. Hydroxypropylcellulose is be added for viscosity adjustment at 0.2-5.0% of total solution weight. An intermediate barrier membrane (Medifilm 390, Mylan) is coated with a skin-contact adhesive (National Starch 387-2051), and the adhesive protected by a removable release liner (SCOTCHPAK 1022, 3M Company). A backing film (SCOTCHPAK 9735, 3M Company) is laminated to the membrane with a partial opening to allow for vertical filling with reservoir vehicle. After filling with reservoir vehicle to a loading of 5.0 mg/cm² fentanyl, the opening is laminated to produce a heat sealed reservoir-type transdermal dosage form. The reservoir contains a ratio of 10 parts fentanyl to 1 part total Antagonist, with the ratio of antagonists being 1 part naloxone HCl to 1 part naltrexone. The transdermal dosage form is useful for treating or preventing pain in an animal, while preventing or discouraging the abuse of the Opioid.

### Example 6

Fentanyl free base (10 g) is added to ethanol (20 g) with mixing. Naloxone HCl (8g) is added to water (37.5g) and propylene glycol (7.5g) with mixing until dissolved. To the naloxone HCl solution is added naltrexone (2g) in ethanol (15 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9726, 3M Company) is coated with a skin-contact adhesive (Solutia 737), and the adhesive protected by a removable release liner (SCOTCHPAK 1022, 3M Company). A backing film (SCOTCHPAK 9735, 3M Company) is laminated to the membrane with a partial opening to allow for vertical filling with reservoir vehicle. After filling with reservoir vehicle to a loading of 2.5 mg/cm² fentanyl, the opening is laminated to produce a heat sealed reservoir-type transdermal dosage form. The reservoir contains a ratio of 1 part fentanyl to 1 part total Antagonist, with the ratio of antagonists being 4 parts naloxone HCl to 1 part naltrexone. The transdermal dosage form is useful for treating or preventing pain in an animal, while preventing or discouraging the abuse of the Opioid.

### Example 7

Fentanyl free base (10 g) is added to ethanol (25 g) with mixing. Naloxone HCl (2g) is added to water (25g) and propylene glycol (5g) with mixing until dissolved. To the naloxone HCl solution is added naltrexone (8g) in ethanol (25 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9726, 3M Company) is coated with a skin-contact adhesive (Solutia 737), and the adhesive protected by a removable release liner (SCOTCHPAK 1022, 3M Company). A backing film (SCOTCHPAK 9735, 3M Company) is laminated to the membrane with a partial opening to allow for vertical filling with reservoir vehicle. After filling with reservoir vehicle to a loading of 2.5 mg/cm² fentanyl, the opening is laminated to produce a heat sealed reservoir-type transdermal dosage form. The reservoir contains a ratio of 1 part fentanyl to 1 part total Antagonist, with the ratio of antagonists being 1 part naloxone HCl to 4 parts naltrexone. The transdermal dosage form is useful for treating or preventing pain in an animal, while preventing or discouraging the abuse of the Opioid.

### Example 8

Fentanyl free base (5.0 g) is added to ethanol (15 g) with mixing. Naloxone HCl (0.9g) is added to water (59g) and propylene glycol (10g) with mixing until dissolved. To the naloxone HCl solution is added naltrexone (0.1g) in ethanol (10 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9726, 3M Company) is coated with a skin-contact adhesive (Solutia 737), and the adhesive protected by a removable release liner (SCOTCHPAK 1022, 3M Company). After filling with reservoir vehicle to a loading of 5.0 mg/cm² fentanyl, a backing film (SCOTCHPAK 9735, 3M Company) is laminated to the membrane to produce a heat sealed reservoir-type transdermal dosage form. The reservoir contains a ratio of 5 parts fentanyl to 1 part total Antagonist, with the ratio of antagonists being 9 parts naloxone HCl to 1 part naltrexone. The transdermal dosage form is useful for treating or preventing pain in an animal, while preventing or discouraging the abuse of the Opioid.

### Example 9

Fentanyl free base (7.5 g) is added to ethanol (12.5 g) with mixing until dissolved. Naloxone HCl (0.25g) is added to water (68g) and propylene glycol (3g) with mixing until dissolved. To the naloxone HCl solution is added naltrexone (1.25g) in ethanol (7.5 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9726, 3M Company) is coated with a skin-contact adhesive (Solutia 737), and the adhesive protected by a removable release liner (SCOTCHPAK 1022, 3M Company). After filling with reservoir vehicle to a loading of 5.0 mg/cm² fentanyl, a backing film (SCOTCHPAK 9735, 3M Company) is laminated to the membrane to produce a heat sealed reservoir-type transdermal dosage form. The reservoir contains a ratio of 5 parts fentanyl to 1 part total Antagonist, with the ratio of antagonists being 1 part naloxone HCl to 5 parts naltrexone. The transdermal dosage form is useful for treating or preventing pain in an animal, while preventing or discouraging the abuse of the Opioid.

### Example 10

Fentanyl free base (5.0 g) and lauric acid (2.5g) are added to ethanol (15 g) with mixing until dissolved. Naloxone HCl (2.5g) is added to water (45g) with mixing until dissolved. To the naloxone HCl solution is added naltrexone (2.5g) in ethanol (15 g). The resulting solution is combined with the fentanyl solution to form the reservoir vehicle. Hydroxypropylcellulose is be added for viscosity adjustment at 0.2-5.0% of total solution weight. An intermediate barrier membrane (COTRAN 9702, 3M Company) is coated with a skin-contact adhesive (Solutia 737), and the adhesive protected by a removable release liner (SCOTCHPAK 1022, 3M Company). After filling with reservoir vehicle to a loading of 5.0 mg/cm² fentanyl, a backing film (SCOTCHPAK 9735, 3M Company) is laminated to the membrane to produce a heat sealed reservoir-type transdermal dosage form. The reservoir contains a ratio of 1 part fentanyl to 1 part total Antagonist, with the ratio of antagonists being 1 part naloxone HCl to 1 part naltrexone. The transdermal dosage form is useful for treating or preventing pain in an animal, while preventing or discouraging the abuse of the Opioid.

### Example 11

Fentanyl free base (10 g) is added to methanol (90g) with mixing. Naloxone free base (5g) is dissolved in methanol (25g) and naltrexone HCl (5g) is dissolved in water (25g), the two solutions combined, and added to the fentanyl solution. A solvent based pressure sensitive adhesive (Dow Silicones #7-4400) is added to the solution in sufficient quantity to produce a combined solution containing, by weight solids, 90% adhesive solids, 5% fentanyl, 2.5% naloxone free base and 2.5% naltrexone HCl. The fentanyl-adhesive mixture is cast onto a release liner (SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 1 part fentanyl to 1 part total Antagonist, with the ratio of antagonists being 1 part naloxone free base to 1 part naltrexone HCl. The transdermal dosage form is useful for treating or preventing pain in an animal, while preventing or discouraging the abuse of the Opioid.

### Example 12

Fentanyl free base (10 g) is added to methanol (90 g) with mixing. Naloxone free base (0.5g) is dissolved in methanol (25g) and naltrexone HCl (0.5g) is dissolved in water (25g), the two solutions combined, and added to the fentanyl solution. A solvent based pressure sensitive adhesive (Dow Silicones #7-4400) is added to the solution in sufficient quantity to produce a combined solution containing, by weight solids, 94% adhesive solids, 5% fentanyl, 0.25% naloxone free base and 0.25% naltrexone HCl. The fentanyl-adhesive mixture is cast onto a release liner SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 10 parts fentanyl to 1 part total Antagonist, with the ratio of antagonists being 1 part naloxone free base to 1 part naltrexone HCl. The transdermal dosage form is useful for treating or preventing pain in an animal, while preventing or discouraging the abuse of the Opioid.

### Example 13

Fentanyl free base (10g) is added to methanol (90 g) with mixing. Naloxone free base (8g) is dissolved in methanol (45g) and naltrexone HCl (2g) is dissolved in water (25g), the two solutions combined, and added to the fentanyl solution. A solvent based pressure sensitive adhesive (Solutia 737) is added to the solution in sufficient quantity to produce a combined solution containing, by weight solids, 90% adhesive solids, 5% fentanyl, 4% naloxone free base and 1% naltrexone HCl. The fentanyl-adhesive mixture is cast onto a release liner (SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 1 part fentanyl to 1 part total Antagonist, with the ratio of antagonists being 4 parts naloxone free base to 1 part naltrexone HCl. The transdermal dosage form is useful for treating or preventing pain in an animal, while preventing or discouraging the abuse of the Opioid.

### Example 14

Fentanyl free base (10 g) is added to methanol (90 g) with mixing. Naloxone free base (2g) is dissolved in methanol (25g) and naltrexone HCl (8g) is dissolved in water (45g), the two solutions combined, and added to the fentanyl solution. A solvent based pressure sensitive adhesive (Solutia 737) is added to the solution in sufficient quantity to produce a combined solution containing, by weight solids, 90% adhesive solids, 5% fentanyl, 1% naloxone free base and 4% naltrexone HCl. The fentanyl-adhesive mixture is cast onto a release liner (SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 1 part fentanyl to 1 part total Antagonist, with the ratio of antagonists being 1 part naloxone free base to 4 parts naltrexone HCl. The transdermal dosage form is useful for treating or preventing pain in an animal, while preventing or discouraging the abuse of the Opioid.

### Example 15

Fentanyl free base (10 g) is added to methanol (90 g) with mixing. Naloxone free base (1.8g) is dissolved in methanol (20g) and naltrexone HCl (0.2g) is dissolved in water (15g), the two solutions combined, and added to the fentanyl solution. A solvent based pressure sensitive adhesive (Solutia 737) is added to the solution in sufficient quantity to produce a combined solution containing, by weight solids, 94% adhesive solids, 5% fentanyl, 0.9% naloxone free base and 0.1% naltrexone HCl. The fentanyl-adhesive mixture is cast onto a release liner (SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 5 parts fentanyl to 1 part total Antagonist, with the ratio of antagonists being 9 parts naloxone free base to 1 part naltrexone HCl. The transdermal dosage form is useful for treating or preventing pain in an animal, while preventing or discouraging the abuse of the Opioid.

### Example 16

Fentanyl free base (7.5 g) is added to methanol (90 g) with mixing. Naloxone free base (0.25g) is dissolved in methanol (20g) and naltrexone HCl (1.25g) is dissolved in water (20g), the two solutions combined, and added to the fentanyl solution. A solvent based pressure sensitive adhesive (Solutia 737) is added to the solution in sufficient quantity to produce a combined solution containing, by weight solids, 91 % adhesive solids, 7.5% fentanyl, 0.25% naloxone free base and 1.25% naltrexone HCl. The fentanyl-adhesive mixture is cast onto a release liner (SCOTCHPAK 1022, 3M Company), and dried to remove solvents. The coating layer contains a fentanyl free base loading of 15mg/cm². A polyethylene film overlay backing tape (SCOTCHPAK 9732, 3M Company) is heat laminated to the upper surface of the adhesive layer. The resulting transdermal dosage form contains a ratio of 5 parts fentanyl to 1 part total Antagonist, with the ratio of antagonists being 1 part naloxone free base to 5 parts naltrexone HCl. The transdermal dosage form is useful for treating or preventing pain in an animal, while preventing or discouraging the abuse of the Opioid.

### Example 17

Fentanyl free base (10 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naloxone HCl (5g) is dissolved in water (25g) and nalmefene free base (5g) is dissolved in methanol (25g), the 2 solutions combined, and added to an adhesive mixture as described above, to form a naloxone HCl-nalmefene-adhesive mixture containing, by weight solids, 90% adhesive solids, 5% nalmefene free base and 5% naloxone HCl.

The fentanyl-adhesive mixture is cast onto a release liner SCOTCHPAK 1022, 3M Company), and dried to remove solvents. The coating layer contains a fentanyl free base loading of 15mg/cm². The naloxone HCl-nalmefene-adhesive mixture is cast onto a barrier membrane (SCOTCHPAK 1006, 3M Company), and dried to remove solvents. The naloxone HCl-nalmefene-adhesive mixture coating contains a combined naloxone HCl and nalmefene loading of 15mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 1 part fentanyl to 1 part total Antagonist, with the ratio of antagonists being 1 part naloxone HCl to 1 part nalmefene. The transdermal dosage form is useful for treating or preventing pain in an animal, while preventing or discouraging the abuse of the Opioid.

### Example 18

Fentanyl free base (10 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naloxone HCl (0.5g) is dissolved in water (20g) and nalmefene free base (0.5g) is dissolved in methanol (20g), the 2 solutions combined, and added to an adhesive mixture as described above, to form a naloxone HCl-nalmefene-adhesive mixture containing, by weight solids, 99% adhesive solids, 0.5% nalmefene free base and 0.5% naloxone HCl.

The fentanyl-adhesive mixture is cast onto a release liner (SCOTCHPAK 1022, 3M Company), and dried to remove solvents. The coating layer contains a fentanyl free base loading of 13.5mg/cm². The naloxone HCl-nalmefene-adhesive mixture is cast onto a barrier membrane (SCOTCHPAK 1006, 3M Company), and dried to remove solvents. The naloxone HCl-nalmefene-adhesive mixture coating contains a combined naloxone HCl and nalmefene loading of 1.5mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 10 part fentanyl to 1 part total Antagonist, with the ratio of antagonists being 1 part naloxone HCl to 1 part nalmefene. The transdermal dosage form is useful for treating or preventing pain in an animal, while preventing or discouraging the abuse of the Opioid.

### Example 19

Fentanyl free base (10 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naltrexone HCl (5g) is dissolved in water (25g) and nalmefene free base (5g) is dissolved in methanol (25g), the 2 solutions combined, and added to an adhesive mixture as described above, to form a naltrexone HCl-nalmefene-adhesive mixture containing, by weight solids, 90% adhesive solids, 5% nalmefene free base and 5% naltrexone HCl.

The fentanyl-adhesive mixture is cast onto a release liner SCOTCHPAK 1022, 3M Company), and dried to remove solvents. The coating layer contains a fentanyl free base loading of 15mg/cm². The naltrexone HCl-nalmefene-adhesive mixture is cast onto a barrier membrane (SCOTCHPAK 1006, 3M Company), and dried to remove solvents. The naltrexone HCl-nalmefene-adhesive mixture coating contains a combined naltrexone HCl and nalmefene loading of 15mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 1 part fentanyl to 1 part total Antagonist, with the ratio of antagonists being 1 part naltrexone HCl to 1 part nalmefene. The transdermal dosage form is useful for treating or preventing pain in an animal, while preventing or discouraging the abuse of the Opioid.

### Example 20

Fentanyl free base (10 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naltrexone HCl (0.5g) is dissolved in water (20g) and nalmefene free base (0.5g) is dissolved in methanol (20g), the 2 solutions combined, and added to an adhesive mixture as described above, to form a naltrexone HCl-nalmefene-adhesive mixture containing, by weight solids, 99% adhesive solids, 0.5% nalmefene free base and 0.5% naltrexone HCl.

The fentanyl-adhesive mixture is cast onto a release liner SCOTCHPAK 1022, 3M Company), and dried to remove solvents. The coating layer contains a fentanyl free base loading of 13.5mg/cm². The naltrexone HCl-nalmefene-adhesive mixture is cast onto a barrier membrane (SCOTCHPAK 1006, 3M Company), and dried to remove solvents. The naltrexone HCl-nalinefene-adhesive mixture coating contains a combined naltrexone HCl and nalmefene loading of 1.5mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 10 parts fentanyl to 1 part total Antagonist, with the ratio of antagonists being 1 part naltrexone HCl to 1 part nalmefene. The transdermal dosage form is useful for treating or preventing pain in an animal, while preventing or discouraging the abuse of the Opioid.

### Example 21

Fentanyl free base (5.0 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naloxone HCl (0.9g) is dissolved in water (20g) and nalmefene free base (0.1g) is dissolved in methanol (20g), the 2 solutions combined, and added to an adhesive mixture as described above, to form a naloxone HCl-nalmefene-adhesive mixture containing, by weight solids, 99% adhesive solids, 0.9% nalmefene free base and 0.1 % naloxone HCl.

The fentanyl-adhesive mixture is cast onto a release liner SCOTCHPAK 1022, 3M Company), and dried to remove solvents. The coating layer contains a fentanyl free base loading of 12mg/cm². The naloxone HCl-nalmefene-adhesive mixture is cast onto a barrier membrane (SCOTCHPAK 1006, 3M Company), and dried to remove solvents. The naloxone HCl-nalmefene-adhesive mixture coating contains a combined naloxone HCl and nalmefene loading of 3mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 5 parts fentanyl to 1 part total Antagonist, with the ratio of antagonists being 9 parts naloxone HCl to 1 part nalmefene. The transdermal dosage form is useful for treating or preventing pain in an animal, while preventing or discouraging the abuse of the Opioid.

### Example 22

Fentanyl free base (7.5 g) is added to methanol (75 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naloxone HCl (0.25g) is dissolved in water (20g) and nalmefene free base (1.25g) is dissolved in methanol (20g), the 2 solutions combined, and added to an adhesive mixture as described above, to form a naloxone HCl-nalmefene-adhesive mixture containing, by weight solids, 97.75% adhesive solids, 0.375% nalmefene free base and 1.875% naloxone HCl.

The fentanyl-adhesive mixture is cast onto a release liner SCOTCHPAK 1022, 3M Company), and dried to remove solvents. The coating layer contains a fentanyl free base loading of 15mg/cm². The naloxone HCl-nalmefene-adhesive mixture is cast onto a barrier membrane (SCOTCHPAK 1006, 3M Company), and dried to remove solvents. The naloxone HCl-nalmefene-adhesive mixture coating contains a combined naloxone HCl and nalmefene loading of 3mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 5 parts fentanyl to 1 part total Antagonist, with the ratio of antagonists being 1 part naloxone HCl to 5 parts nalmefene. The transdermal dosage form is useful for treating or preventing pain in an animal, while preventing or discouraging the abuse of the Opioid.

### Example 23

Fentanyl free base (10 g) is added to methanol (90 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naltrexone HCl (0.2g) is dissolved in water (20g) and nalmefene free base (1.8g) is dissolved in methanol (20g), the 2 solutions combined, and added to an adhesive mixture as described above, to form a naltrexone HCl-nalmefene-adhesive mixture containing, by weight solids, 98% adhesive solids, 1.8% nalmefene free base and 0.2% naltrexone HCl.

The fentanyl-adhesive mixture is cast onto a release liner (SCOTCHPAK 1022, 3M Company), and dried to remove solvents. The coating layer contains a fentanyl free base loading of 15mg/cm². The naltrexone HCl-nalmefene-adhesive mixture is cast onto a barrier membrane (SCOTCHPAK 1006, 3M Company), and dried to remove solvents. The naltrexone HCl-nalmefene-adhesive mixture coating contains a combined naltrexone HCl and nalmefene loading of 3mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 5 parts fentanyl to 1 part total Antagonist, with the ratio of antagonists being 1 part naltrexone HCl to 9 parts nalmefene. The transdermal dosage form is useful for treating or preventing pain in an animal, while preventing or discouraging the abuse of the Opioid.

### Example 24

Fentanyl free base (7.5 g) is added to methanol (75 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naloxone HCl (0.9g) is dissolved in water (20g) and nalmefene free base (0.1g) is dissolved in methanol (20g), the 2 solutions combined, and added to an adhesive mixture as described above, to form a naloxone HCl-nalmefene-adhesive mixture containing, by weight solids, 99% adhesive solids, 0.1% nalmefene free base and 0.9% naloxone HCl.

The fentanyl-adhesive mixture is cast onto a release liner (SCOTCHPAK 1022, 3M Company), and dried to remove solvents. The coating layer contains a fentanyl free base loading of 15mg/cm². The naloxone HCl-nalmefene-adhesive mixture is cast onto a barrier membrane (SCOTCHPAK 1006, 3M Company), and dried to remove solvents. The naloxone HCl-nalmefene-adhesive mixture coating contains a combined naloxone HCl and nalmefene loading of 1.0mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 15 parts fentanyl to 1 part total Antagonist, with the ratio of antagonists being 9 parts naloxone HCl to 1 part nalmefene. The transdermal dosage form is useful for treating or preventing pain in an animal, while preventing or discouraging the abuse of the Opioid.

### Example 25

Fentanyl free base (21 g) is added to methanol (150 g) with mixing. A solvent based pressure sensitive adhesive (Dow Silicones #7-4402) is added to the solution in sufficient quantity to produce a combined fentanyl-adhesive mixture containing, by weight solids, 90% adhesive solids and 10% fentanyl.

Naltrexone HCl (2g) is dissolved in water (20g) and nalmefene free base (5g) is dissolved in methanol (25g), the 2 solutions combined, and added to an adhesive mixture as described above, to form a naltrexone HCl-nalmefene-adhesive mixture containing, by weight solids, 95.975% adhesive solids, 2.875% nalmefene free base and 1.15% naltrexone HCl.

The fentanyl-adhesive mixture is cast onto a release liner (SCOTCHPAK 1022, 3M Company), and dried to remove solvents. The coating layer contains a fentanyl free base loading of 12mg/cm². The naltrexone HCl-nalmefene-adhesive mixture is cast onto a barrier membrane (SCOTCHPAK 1006, 3M Company), and dried to remove solvents. The naltrexone HCl-nalmefene-adhesive mixture coating contains a combined naltrexone HCl and nalmefene loading of 4mg/cm². The barrier membrane is next laminated onto the release liner so that the fentanyl-containing layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 3 parts fentanyl to 1 part total Antagonist, with the ratio of antagonists being 1 part naltrexone HCl to 2.5 parts nalmefene. The transdermal dosage form is useful for treating or preventing pain in an animal, while preventing or discouraging the abuse of the Opioid.

### Example 26

Fentanyl free base (6 g) is added to methanol (65 g) with mixing. A solvent based pressure sensitive adhesive (Dow Silicones #7-4402) is added to the solution in sufficient quantity to produce a combined fentanyl-adhesive mixture containing, by weight solids, 88% adhesive solids and 12% fentanyl.

Naltrexone HCl (0.33g) is dissolved in water (15g) and nalmefene free base (0.67g) is dissolved in methanol (20g), the 2 solutions combined, and added to an adhesive mixture as described above, to form a naltrexone HCl-nalmefene-adhesive mixture containing, by weight solids, 97% adhesive solids, 2% nalmefene free base and 1% naltrexone HCl.

The fentanyl-adhesive mixture is cast onto a release liner (SCOTCHPAK 1022, 3M Company), and dried to remove solvents. The coating layer contains a fentanyl free base loading of 12mg/cm². The naltrexone HCl-nalmefene-adhesive mixture is cast onto a barrier membrane (SCOTCHPAK 1006, 3M Company), and dried to remove solvents. The naltrexone HCl-nalmefene-adhesive mixture coating contains a combined naltrexone HCl and nalmefene loading of 2mg/cm². The barrier membrane is next laminated onto the release liner so that the fentanyl-containng layer is separated from the Antagonist-containing layer by the barrier membrane. The resulting 2-layer coated liner is laminated onto a polyester film overlay (SCOTCHPAK 9732, available from the 3M Company). The transdermal dosage form contains a ratio of 6 parts fentanyl to 1 part total Antagonist, with the ratio of antagonists being 1 part naltrexone HCl to 2 parts nalmefene. The transdermal dosage form is useful for treating or preventing pain in an animal, while preventing or discouraging the abuse of the Opioid.

### Example 27

A Fentanyl 15mg/cm² transdermal device is prepared according to Examples 17-26. In this example, however, the Antagonist-adhesive layer is prepared and incorporated into the transferal dosage form as two separate, but adjoining layers, a first layer containing a naloxone HCl-adhesive mixture, with a naloxone HCl loading of 7.5mg/cm²; and a second layer containing a nalmefene-adhesive mixture, with a nalmefene loading of 7.5mg/cm². The transdermal dosage forms are useful for treating or preventing pain in an animal, while preventing or discouraging the abuse of the Opioid.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

## Claims

1. A transdermal dosage form comprising:
an active agent or a pharmaceutically acceptable salt thereof;
an antagonist in the form of a free base; and
an antagonist in the form of a pharmaceutically acceptable salk

2. The transdermal dosage form of claim 1, wherein the antagonist in the form of a free base is in an amount sufficient to inhibit at least one biological effect of the active agent or pharmaceutically acceptable salt thereof.

3. The transdermal dosage form of claim 1, wherein the pharmaceutically acceptable salt of the antagonist is in an amount sufficient to inhibit at least one biological effect of the active agent or pharmaceutically acceptable salt thereof.

4. The transdermal dosage form of claim 1, wherein both the antagonist in the form of a free base and the pharmaceutically acceptable salt of the antagonist are in an amount sufficient to inhibit at least one biological effect of the active agent or acceptable salt thereof.

5. The transdermal dosage form of claim 1, wherein the pharmaceutically acceptable salt of the antagonist and the antagonist in the form of a free base are based on the same antagonist.

6. The transdermal dosage form of claim 1, wherein the amount of the active agent or a pharmaceutically acceptable salt thereof, is from about 0,1 to about 500 mg and the weight ratio of the active agent, or pharmaceutically acceptable salt thereof, to the total amount of antagonist in the form of a free base and pharmaceutically acceptable salt of an antagonist is from about 15:1 to about 1:5.

7. The transdermal dosage form of claim 1, wherein the amount of the active agent or a pharmaceutically acceptable salt thereof, is from about 0.1 to about 500 mg and the weight ratio of the active agent, or pharmaceutically acceptable salt thereof, to the total amount of antagonist in the form of a free base and pharmaceutically acceptable salt of an antagonist is from about 12:1 to about 4:1.

8. The transdermal dosage form of claim 1, wherein the transdermal dosage form comprises a reservoir comprising the active agent, or a pharmaceutically acceptable salt thereof, the antagonist in the form of a free base and the pharmaceutically acceptable salt of an antagonist.

9. The transdermal dosage form of claim 1, wherein the transdermal dosage form is a polymer-matrix-type transdermal dosage form.

10. The transdermal dosage form of claim 1, wherein the transdermal dosage form is a drug-in-adhesive-type transdermal dosage form.

11. The transdermal dosage form of claim 1, wherein the active agent is an opioid or a pharmaceutically acceptable salt thereof; and both the antagonist in the form of a free base and the pharmaceutically acceptable salt of an antagonist are opioid antagonists.

12. The transdermal dosage form of claim 11, wherein the opioid antagonist in the form of a free base is in an amount sufficient to inhibit the euphoric effect of the opioid or pharmaceutically acceptable salt thereof.

13. The transdermal dosage form of claim 11, wherein the pharmaceutically acceptable salt of the opioid antagonist is in an amount sufficient to inhibit the euphoric effect of the opioid or pharmaceutically acceptable salt thereof.

14. The transdermal dosage form of claim 11, wherein both the opioid antagonist in the form of a free base and the pharmaceutically acceptable salt of the opioid antagonist are in an amount sufficient to inhibit the euphoric effect of the opioid or pharmaceutically acceptable salt thereof.

15. The transdermal dosage form of claim 11, wherein the pharmaceutically acceptable salt of the opioid antagonist and the opioid antagonist in the form of a free base are based on the same opioid antagonist.

16. The transdermal dosage form of claim 11, wherein the amount of the opioid or a pharmaceutically acceptable salt thereof is from about 0.1 to about 500 mg and the weight ratio of the opioid, or pharmaceutically acceptable salt thereof, to the total amount of opioid antagonist in the form of a free base and pharmaceutically acceptable salt of an opioid antagonist is from about 15:1 to about 1:5.

17. The transdermal dosage form of claim 11, wherein the amount of the opioid or a pharmaceutically acceptable salt thereof, is from about 0.1 to about 500 mg and the weight ratio of the opioid, or pharmaceutically acceptable salt thereof, to the total amount of opioid antagonist in the form of a free base and pharmaceutically acceptable salt of an opioid antagonist is from about 12:1 to about 4:1.

18. The transdermal dosage form of claim 11, wherein the transdermal dosage form comprises a reservoir comprising the opioid, or a pharmaceutically acceptable salt thereof, the opioid antagonist in the form of a free base and the pharmaceutically acceptable salt of an opioid antagonist.

19. The transdermal dosage form of claim 11, wherein the transdermal dosage form is a polymer-matrix-type transdermal dosage form.

20. The transdermal dosage form of claim 11, wherein the transdermal dosage form is a drug-in-adhesive-type transdermal dosage form.

21. The transdermal dosage form of claim 11, wherein the opioid or pharmaceutically acceptable salt thereof is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, pharmaceutically acceptable salts thereof and mixtures of any two or more thereof.

22. The transdermal dosage form of claim 21, wherein the opioid or pharmaceutically acceptable salt thereof is oxycodone or a pharmaceutically acceptable salt thereof.

23. The transdermal dosage form of claim 21, wherein the opioid or pharmaceutically acceptable salt thereof is hydrocodone or a pharmaceutically acceptable salt thereof.

24. The transdermal dosage form of claim 21, wherein the opioid or pharmaceutically acceptable salt thereof is buprenorphine or a pharmaceutically acceptable salt thereof.

25. The transdermal dosage form of claim 21, wherein the opioid or pharmaceutically acceptable salt thereof is fentanyl or a pharmaceutically acceptable salt thereof.

26. The transdermal dosage form of claim 25, wherein the opioid antagonist in the form of a free base is naltrexone and the pharmaceutically acceptable salt is naloxone HCl.

27. The transdermal dosage form of claim 25, wherein the opioid antagonist in the form of a free base is naloxone and the pharmaceutically acceptable salt is naltrexone HCl.

28. The transdermal dosage form of claim 25, wherein the opioid antagonist in the form of a free base is nalmefene and the pharmaceutically acceptable salt is naloxone HCL

29. The transdermal dosage form of claim 25, wherein the opioid antagonist in the form of a free base is nalmefene and the pharmaceutically acceptable salt is naltrexone HCl.

30. The transdermal dosage form of claim 11, wherein the opioid antagonist in the form of a free base is selected from the group consisting of cyclazocine, naloxone, naltrexone, nalmefene, nalbuphine, nalorphine, cyclazacine and levallorphan.

31. The transdermal dosage form of claim 30, wherein the opioid antagonist in the form of a free base is selected from the group consisting of naloxone, naltrexone and nalmefene.

32. The transdermal dosage form of claim 11, wherein the pharmaceutically acceptable salt of an opioid antagonist is a pharmaceutically acceptable salt of an opioid antagonist selected from the group consisting of cyclazocine, naloxone, naltrexone, nalmefene, nalbuphine, nalorphine, cyclazacine and levallorphan.

33. The transdermal dosage form of claim 32, wherein the pharmaceutically acceptable salt of an opioid antagonist is a pharmaceutically acceptable salt of an opioid antagonist selected from the group consisting of naloxone, naltrexone and nalmefene.

34. A kit for treating pain in a patient, comprising:
a) the transdermal-delivery device of claim 11; and
b) a printed set of instructions directing the use of the transdermal dosage form to treat pain.

35. Use of an opioid or a pharmaceutically acceptable salt thereof, an opioid antagonist in the form of a free base, and an opioid antagonist in the form of a pharmaceutically acceptable salt for the manufacturing of a transdermal dosage form for treating or preventing pain in a patient.

## Patentansprüche

1. Eine transdermale Verabreichungsform umfassend:
einen Wirkstoff oder ein pharmazeutisch annehmbares Salz davon,
einen Antagonisten in der Form einer freien Base, und ein
ein Antagonist in Form eines pharmazeutisch annehmbaren Salzes.

2. Die transdermale Verabreichungsform nach Anspruch 1, worin der Antagonist in der Form einer freien Base in einer Menge vorhanden ist, die ausreicht, um zumindest einen biologischen Effekt des Wirkstoffes oder des pharmazeutisch annehmbaren Salzes davon zu inhibieren.

3. Die transdermale Verabreichungsform nach Anspruch 1, worin das pharmazeutisch annehmbare Salz des Antagonisten in einer Menge vorhanden ist, die ausreicht, um zumindest einen biologischen Effekt des Wirkstoffes oder des pharmazeutisch annehmbaren Salzes davon zu inhibieren.

4. Die transdermale Verabreichungsform nach Anspruch 1, worin beide, der Antagonist in der Form einer freien Base und das pharmazeutisch annehmbare Salz des Antagonisten, in einer Menge vorhanden sind, ausreichend um zumindest einen biologischen Effekt des Wirkstoffes oder des annehmbaren Salzes davon zu inhibieren.

5. Die transdermale Verabreichungsform nach Anspruch 1, worin das pharmazeutisch annehmbare Salz des Antagonisten und der Antagonist in der Form einer freien Base auf demselben Antagonisten basieren.

6. Die transdermale Verabreichungsform durch Anspruch 1, worin die Menge des Wirkstoffs oder eines pharmazeutisch annehmbaren Salzes davon etwa 0,1 bis etwa 500 mg beträgt und das Gewichtsverhältnis des Wirkstoffs oder des pharmazeutisch annehmbaren Salzes davon zur Gesamtmenge des Antagonisten in der Form einer freien Base und des pharmazeutisch annehmbaren Salzes des Antagonisten etwa 15:1 bis etwa 1:5 beträgt.

7. Die transdermale Verabreichungsform durch Anspruch 1, worin die Menge des Wirkstoffs oder eines pharmazeutisch annehmbaren Salzes davon etwa 0,1 bis etwa 500 mg beträgt und das Gewichtsverhältnis des Wirkstoffs oder des pharmazeutisch annehmbaren Salzes davon zur Gesamtmenge des Antagonisten in der Form einer freien Base und des pharmazeutisch annehmbaren Salzes des Antagonisten von etwa 12:1 bis etwa 4:1 ist.

8. Die transdermale Verabreichungsform nach Anspruch 1, worin die transdermale Verabreichungsform ein Reservoir umfasst, welches den Wirkstoff oder ein pharmazeutisch annehmbares Salz davon, den Antagonisten in der Form einer freien Base und das pharmazeutisch annehmbare Salz eines Antagonisten umfasst.

9. Die transdermale Verabreichungsform nach Anspruch 1, worin die transdermale Verabreichungsform eine transdermale Verabreichungsform des Polymer-Matrix-Typs ist.

10. Die transdermale Verabreichungsform nach Anspruch 1, worin die transdermale Verabreichungsform eine transdermale Verabreichungsform des Arzneimittelin-Haftmittel-Typs ist.

11. Die transdermale Verabreichungsform nach Anspruch 1, worin der Wirkstoff ein Opioid oder ein pharmazeutisch annehmbares Salz davon ist und beide, der Antagonist in der Form der freien Base und das pharmazeutisch annehmbare Salz des Antagonisten, Opioidantagonisten sind.

12. Die transdermale Verabreichungsform nach Anspruch 11, worin der Opioidantagonist in der Form einer freien Base in einer Menge vorhanden ist, die ausreicht, um den euphorischen Effekt des Opioides oder des pharmazeutisch annehmbaren Salzes davon zu inhibieren.

13. Die transdermale Verabreichungsform nach Anspruch 11, worin das pharmazeutisch annehmbare Salz des Opioidantagonisten in einer Menge vorhanden ist, die ausreicht, um den euphorischen Effekt des Opioids oder des pharmazeutisch annehmbaren Salzes davon zu inhibieren.

14. Die transdermale Verabreichungsform nach Anspruch 11, worin beide, der Opioidantagonist in der Form einer freien Base und das pharmazeutisch annehmbare Salz des Opioidantagonisten, in Mengen vorhanden sind, ausreichend den euphorischen Effekt des Opioids oder pharmazeutisch annehmbaren Salzes davon zu inhibieren.

15. Die transdermale Verabreichungsform nach Anspruch 11, worin das pharmazeutisch annehmbare Salz des Opioidantagonisten und der Opioidantagonist in der Form einer freien Base auf demselben Opioidantagonisten basieren.

16. Die transdermale Verabreichungsform nach Anspruch 11, worin die Menge des Opioids oder eines pharmazeutisch annehmbaren Salzes davon etwa 0,1 bis etwa 500 mg beträgt und das Gewichtsverhältnis des Opioides oder des pharmazeutisch annehmbaren Salzes davon zur Gesamtmenge des Opioidantagonisten in der Form einer freien Base und des pharmazeutisch annehmbaren Salzes eines Opioidantagonisten von etwa 15:1 zu etwa 1:5 ist.

17. Die transdermale Verabreichungsform nach Anspruch 11, worin die Menge des Opioids oder eines pharmazeutisch annehmbaren Salzes davon etwa 0,1 bis etwa 500 mg beträgt und das Gewichtsverhältnis des Opioides oder des pharmazeutisch annehmbaren Salzes davon zur Gesamtmenge des Opioidantagonisten in Form einer freien Base und des pharmazeutisch annehmbaren Salzes eines Opioidantagonisten von etwa 12:1 zu etwa 4:1 ist.

18. Die transdermale Verabreichungsform nach Anspruch 11, worin die transdermale Verabreichungsform ein Reservoir umfasst, welches das Opioid oder ein pharmazeutisch annehmbares Salz davon, den Opioidantagonisten in der Form einer freien Base und das pharmazeutisch annehmbare Salz eines Opioidantagonisten umfasst.

19. Die transdermale Verabreichungsform nach Anspruch 11, worin die Verabreichungsform eine transdermale Verabreichungsform des Polymer-Matrix-Typs ist.

20. Die transdermale Verabreichungsform nach Anspruch 11, worin die transdermale Verabreichungsform eine transdermale Verabreichungsform des Arzneimittelin-Haftmittel-Typs ist.

21. Die transdermale Verabreichungsform nach Anspruch 11, worin der Opioid oder das pharmazeutisch annehmbare Salz davon ausgewählt ist aus der Gruppe bestehend aus
Alfentanil, Allylprodin, Alphaprodin, Anileridin, Benzylmorphin, Bezitramid, Buprenorphin, Butorphanol, Clonitazen, Codein, Desomorphin, Dextromoramid, Dezocin, Diampromid, Diamorphon, Dihydrocodein, Dihydromorphin, Dihydromorphon, Dihydroisomorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Etorphin, Dihydroetorphin, Fentanyl, Heroin, Hydrocodon, Hydromorphon, Hydromorphandon, Hydroxypethidin, Isomethadon, Ketobernidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorpin, Nalbuphen, Normorphin, Norpipanon, Opium, Oxycodon, Oxymorphon, Pantopon, Papaveretum, Paregoric, Pentazocin, Phenadoxon, Phendimetrazin, Phendimetrazon, Phenomorphan, Phenazocin, Phenoperidin, Piritramid, Propheptazin, Promedol, Properidin, Propoxyphen, Propylhexedrin, Sufentanil, Tilidin, Tramadol, ein pharmazeutisch annehmbares Salz davon oder Mischungen von zweien oder mehreren davon.

22. Die transdermale Verabreichungsform nach Anspruch 21, worin das Opioid oder das pharmazeutisch annehmbare Salz davon Oxycodon oder ein pharmazeutisch annehmbares Salz davon ist.

23. Die transdermale Verabreichungsform nach Anspruch 21, worin das Opioid oder das pharmazeutisch annehmbare Salz davon Hydrocodon oder ein pharmazeutisch annehmbares Salz davon ist.

24. Die transdermale Verabreichungsform nach Anspruch 21, worin das Opioid oder das pharmazeutisch annehmbare Salz davon Buprenorphin oder ein pharmazeutisch annehmbares Salz davon ist.

25. Die transdermale Verabreichungsform nach Anspruch 21, worin das Opioid oder das pharmazeutisch annehmbare Salz davon Fentanyl oder ein pharmazeutisch annehmbares Salz davon ist.

26. Die transdermale Verabreichungsform nach Anspruch 25, worin der Opioidantagonist in der Form einer freien Base Naltrexon und das pharmazeutisch annehmbare Salz Naloxon HCl ist.

27. Die transdermale Verabreichungsform nach Anspruch 25, worin der Opioidantagonist in der Form einer freien Base Naloxon ist und das pharmazeutisch annehmbare Salz Naltrexon HCl ist.

28. Die transdermale Verabreichungsform nach Anspruch 25, worin der Opioidantagonist in der Form einer freien Base Nalmefen und das pharmazeutisch annehmbare Salz davon Naloxon HCl ist.

29. Die transdermale Verabreichungsform nach Anspruch 25, worin der Opioidantagonist in der Form einer freien Base Nalmelfen und das pharmazeutisch annehmbare Salz Naltrexon HCl ist.

30. Die transdermale Verabreichungsform nach Anspruch 11, worin der Opioidantagonist in der Form einer freien Base ausgewählt ist aus der Gruppe bestehend aus Cyclazocin, Naloxon, Naltrexon, Nalmefen, Nalbuphin, Nalorphin, Cyclazin und Levallorphan.

31. Die transdermale Verabreichungsform nach Anspruch 30, worin der Opioidantagonist in der Form einer freien Base ausgewählt wird aus der Gruppe bestehend aus Naloxon, Naltrexon und Nalemefen.

32. Die transdermale Verabreichungsform nach Anspruch 11, worin das pharmazeutisch annehmbare Salz eines Opioidantagonisten ein pharmazeutisch annehmbares Salz eines Opioidantagonisten ist, ausgewählt aus der Gruppe bestehend aus Cyclazocin, Naloxon, Naltrexon, Nalmefen, Nalbuphin, Nalorphin, Cyclazin und Levallorphan.

33. Die transdermale Verabreichungsform nach Anspruch 32, worin das pharmazeutisch annehmbare Salz eines Opioidantagonisten ein pharmazeutisch annehmbares Salz eines Opioidantagonisten ist, ausgewählt aus der Gruppe bestehend aus Naloxon, Naltrexon und Nalmefen.

34. Ein Kit zur Behandlung von Schmerz bei einem Patienten umfassend:
a) die transdermale Verabreichungsform nach Anspruch 11; und
b) ein gedruckter Beipackzettel, welcher auf die Verwendung der transdermalen Verabreichungsform zur Behandlung von Schmerz gerichtet ist.

35. Verwendung eines Opioids oder eines pharmazeutisch annehmbaren Salzes davon, eines Opioidantagonisten in der Form einer Base und
eines Opioidantagonisten in der Form eines pharmazeutisch annehmbaren Salzes für die Herstellung einer transdermalen Verabreichungsform zur Behandlung oder Prävention von Schmerz bei einem Patienten.

## Revendications

1. Forme posologique transdermique, comportant :
un agent actif ou un sel pharmaceutiquement acceptable de celui-ci,
un antagoniste sous la forme d'une base libre, et
un antagoniste sous la forme d'un sel pharmaceutiquement acceptable.

2. Forme posologique transdermique selon la revendication 1, dans laquelle l'antagoniste sous la forme d'une base libre est en une quantité suffisante pour empêcher au moins un effet biologique de l'agent actif, ou d'un sel pharmaceutiquement acceptable de celui-ci.

3. Forme posologique transdermique selon la revendication 1, dans laquelle le sel pharmaceutiquement acceptable de l'antagoniste est en une quantité suffisante pour empêcher au moins un effet biologique de l'agent actif, ou d'un sel pharmaceutiquement acceptable de celui-ci.

4. Forme posologique transdermique selon la revendication 1, dans laquelle l'antagoniste sous la forme d'une base libre et le sel pharmaceutiquement acceptable de l'antagoniste sont en une quantité suffisante pour empêcher au moins un effet biologique de l'agent actif, ou d'un sel acceptable de celui-ci.

5. Forme posologique transdermique selon la revendication 1, dans laquelle le sel pharmaceutiquement acceptable de l'antagoniste et l'antagoniste sous la forme d'une base libre sont basés sur le même antagoniste.

6. Forme posologique transdermique selon la revendication 1, dans laquelle la quantité de l'agent actif, ou d'un sel pharmaceutiquement acceptable de celui-ci, est d'environ 0,1 à environ 500 mg, et le rapport en poids de l'agent actif, ou d'un sel pharmaceutiquement acceptable de celui-ci, par rapport à la quantité totale de l'antagoniste sous la forme d'une base libre et d'un seul pharmaceutiquement acceptable d'un antagoniste est d'environ 15:1 à environ 1:5.

7. Forme posologique transdermique selon la revendication 1, dans laquelle la quantité de l'agent actif ou d'un sel pharmaceutiquement acceptable de celui-ci est d'environ 0,1 à environ 500 mg, et le rapport en poids de l'agent actif, ou d'un sel pharmaceutiquement acceptable de celui-ci, par rapport à la quantité totale d'un antagoniste sous la forme d'une base libre et d'un sel pharmaceutiquement acceptable d'un antagoniste est d'environ 12:1 à environ 4:1.

8. Forme posologique transdermique selon la revendication 1, dans laquelle la forme posologique transdermique comprend un réservoir comprenant l'agent actif, ou un sel pharmaceutiquement acceptable de celui-ci, l'antagoniste sous la forme d'une base libre et le sel pharmaceutiquement acceptable d'un antagoniste.

9. Forme posologique transdermique selon la revendication 1, dans laquelle la forme posologique transdermique est une forme posologique transdermique du type à matrice polymère.

10. Forme posologique transdermique selon la revendication 1, dans laquelle la forme posologique transdermique est une forme posologique transdermique du type médicament dans adhésif.

11. Forme posologique transdermique selon la revendication 1, dans laquelle l'agent actif est un opioïde ou un sel pharmaceutiquement acceptable de celui-ci, et l'antagoniste sous la forme d'une base libre et le sel pharmaceutiquement acceptable d'un antagoniste sont des antagonistes opioïdes.

12. Forme posologique transdermique selon la revendication 11, dans laquelle l'antagoniste opioïde sous la forme d'une base libre est en une quantité suffisante pour empêcher l'effet euphorique de l'opioïde ou d'un sel pharmaceutiquement acceptable de celui-ci.

13. Forme posologique transdermique selon la revendication 11, dans laquelle le sel pharmaceutiquement acceptable de l'antagoniste opioïde est en une quantité suffisante pour empêcher l'effet euphorique de l'opioïde ou d'un sel pharmaceutiquement acceptable de celui-ci.

14. Forme posologique transdermique selon la revendication 11, dans laquelle l'antagoniste opioïde sous la forme d'une base libre et le sel pharmaceutiquement acceptable de l'antagoniste opioïde sont en une quantité suffisante pour empêcher l'effet euphorique de l'opioïde ou d'un sel pharmaceutiquement acceptable de celui-ci.

15. Forme posologique transdermique selon la revendication 11, dans laquelle le sel pharmaceutiquement acceptable de l'antagoniste opioïde et l'antagoniste opioïde sous la forme d'une base libre sont basés sur le même antagoniste opioïde.

16. Forme posologique transdermique selon la revendication 11, dans laquelle la quantité de l'opioïde ou d'un sel pharmaceutiquement acceptable de celui-ci est d'environ 0,1 à environ 500 mg, et le rapport en poids de l'opioïde, ou d'un sel pharmaceutiquement acceptable de celui-ci, sur la quantité totale de l'antagoniste opioïde sous la forme d'une base libre et d'un sel pharmaceutiquement acceptable d'un antagoniste opioïde est d'environ 15:1 à environ 1:5.

17. Forme posologique transdermique selon la revendication 11, dans laquelle la quantité de l'opioïde ou d'un sel pharmaceutiquement acceptable de celui-ci est d'environ 0,1 à environ 500 mg, et le rapport en poids de l'opioïde, ou d'un sel pharmaceutiquement acceptable de celui-ci, sur la quantité totale de l'antagoniste opioïde sous la forme d'une base libre et d'un sel pharmaceutiquement acceptable d'un antagoniste opioïde est d'environ 12:1 à environ 4:1.

18. Forme posologique transdermique selon la revendication 11, dans laquelle la forme posologique transdermique comprend un réservoir comprenant l'opioïde, ou un sel pharmaceutiquement acceptable de celui-ci, l'antagoniste opioïde sous la forme d'une base libre et le sel pharmaceutiquement acceptable d'un antagoniste opioïde.

19. Forme posologique transdermique selon la revendication 11, dans laquelle la forme posologique transdermique est une forme posologique transdermique de type à matrice polymère.

20. Forme posologique transdermique selon la revendication 11, dans laquelle la forme posologique transdermique est une forme posologique transdermique du type médicament dans adhésif.

21. Forme posologique transdermique selon la revendication 11, dans laquelle l'opioïde ou un sel pharmaceutiquement acceptable de celui-ci est sélectionné dans le groupe comprenant alfentanil, allylprodine, alphaprodine, aniléridine, benzylmorphine, bézitramide, buprénorphine, butorphanol, clonitazène, codéine, désomorphine, dextromoramide, dézocine, diampromide, diamorphone, dihydrocodéine, dihydromorphine, dihydromorphone, dihydroisomorphine, diménoxadol, dimépheptanol, diméthylthiambutène, dioxaphétylbutyrate, dipipanone, eptazocine, éthoheptazine, éthylméthylthiambutène, éthylmorphine, étonitazène, étorphine, dihydroétorphine, fentanyl, héroïne, hydrocodone, hydromorphone, hydromorphodone, hydroxypéthidine, isométhadone, cétobémidone, lévorphanol, lévophénacylmorphane, lofentanil, mépéridine, meptazinol, métazocine, méthadone, métopone, morphine, myrophine, narcéine, nicomorphine, norlévorphanol, norméthadone, nalorphine, nalbuphène, normorphine, norpipanone, opium, oxycodone, oxymorphone, pantopone, papaveretum, parégorique, pentazocine, phénadoxone, phendimétrazine, phendimétrazone, phénomorphane, phénazocine, phénopéridine, piminodine, piritramide, propheptazine, promédol, propéridine, propoxyphène, propylhexédrine, sufentanil, tilidine, tramadol, et des sels pharmaceutiquement acceptables de ceux-ci, et des mélanges de deux d'entre eux ou plus.

22. Forme posologique transdermique selon la revendication 21, dans laquelle l'opioïde ou un sel pharmaceutiquement acceptable de celui-ci est de l'oxycodone, ou un sel pharmaceutiquement acceptable de celle-ci.

23. Forme posologique transdermique selon la revendication 21, dans laquelle l'opioïde ou un sel pharmaceutiquement acceptable de celui-ci est de l'hydrocodone, ou un sel pharmaceutiquement acceptable de celle-ci.

24. Forme posologique transdermique selon la revendication 21, dans laquelle l'opioïde ou un sel pharmaceutiquement acceptable de celui-ci est de la buprénorphine, ou un sel pharmaceutiquement acceptable de celle-ci.

25. Forme posologique transdermique selon la revendication 21, dans laquelle l'opioïde ou un sel pharmaceutiquement acceptable de celui-ci est du fentanyl, ou un sel pharmaceutiquement acceptable de celui-ci.

26. Forme posologique transdermique selon la revendication 21, dans laquelle l'antagoniste opioïde sous la forme d'une base libre est de la naltrexone, et le sel pharmaceutiquement acceptable est de la naloxone HCl.

27. Forme posologique transdermique selon la revendication 25, dans laquelle l'antagoniste opioïde sous la forme d'une base libre est de la naloxone, et le sel pharmaceutiquement acceptable est de la naltrexone HCl.

28. Forme posologique transdermique selon la revendication 25, dans laquelle l'antagoniste opioïde sous la forme d'une base libre est du nalméfène, et le sel pharmaceutiquement acceptable est de la naloxone HCl.

29. Forme posologique transdermique selon la revendication 25, dans laquelle l'antagoniste opioïde sous la forme d'une base libre est du nalméfène, et le sel pharmaceutiquement acceptable est de la naltrexone HCl.

30. Forme posologique transdermique selon la revendication 11, dans laquelle l'antagoniste opioïde sous la forme d'une base libre est sélectionné dans le groupe comprenant cyclazocine, naloxone, naltrexone, nalméfène, nalbuphine, nalorphine, cyclazacine et lévallorphane.

31. Forme posologique transdermique selon la revendication 30, dans laquelle l'antagoniste opioïde sous la forme d'une base libre est sélectionné dans le groupe comprenant naloxone, naltrexone et nalméfène.

32. Forme posologique transdermique selon la revendication 11, dans laquelle le sel pharmaceutiquement acceptable d'un antagoniste opioïde est un sel pharmaceutiquement acceptable d'un antagoniste opioïde sélectionné dans le groupe comprenant cyclazocine, naloxone, naltrexone, nalméfène, nalbuphine, nalorphine, cyclazacine et lévallorphane.

33. Forme posologique transdermique selon la revendication 32, dans laquelle le sel pharmaceutiquement acceptable d'un antagoniste opioïde est un sel pharmaceutiquement acceptable d'un antagoniste opioïde sélectionné dans le groupe comprenant naloxone, naltrexone et nalméfène.

34. Kit pour traiter la douleur chez un patient, comportant :
a) le dispositif de délivrance transdermique selon la revendication 11, et
b) un ensemble d'instructions imprimé indiquant l'utilisation de la forme posologique transdermique pour traiter la douleur.

35. Utilisation d'un opioïde ou d'un sel pharmaceutiquement acceptable de celui-ci, et d'un antagoniste opioïde sous la forme d'une base libre, et d'un antagoniste opioïde sous la forme d'un sel pharmaceutiquement acceptable, pour la fabrication d'une forme posologique transdermique pour traiter ou empêcher la douleur chez un patient.
